# EUROPEAN PATENT SPECIFICATION

(11) **EP 2 451 400 B1**
(45) Date of publication and mention of the grant of the patent: **26.10.2016**
(21) Application number: 10797421.4
(22) Date of filing: 12.07.2010
(51) Int. Cl.: A61F 2/32, A61F 2/34, A61F 2/36, A61F 2/30, A61F 2/46

(54) **HIP JOINT DEVICE**
HÜFTGELENKVORRICHTUNG
PROTHÈSE DE HANCHE

(30) Priority: 10.07.2009 SE 0900981; 10.07.2009 SE 0900957; 10.07.2009 SE 0900959; 10.07.2009 SE 0900960; 10.07.2009 SE 0900962; 10.07.2009 SE 0900963; 10.07.2009 SE 0900965; 10.07.2009 SE 0900966; 10.07.2009 SE 0900968; 10.07.2009 SE 0900969; 10.07.2009 SE 0900970; 10.07.2009 SE 0900972; 10.07.2009 SE 0900973; 10.07.2009 SE 0900974; 10.07.2009 SE 0900976; 10.07.2009 SE 0900978; 10.07.2009 SE 0900958; 30.07.2009 US 229738 P; 30.07.2009 US 229739 P; 30.07.2009 US 229743 P; 30.07.2009 US 229745 P; 30.07.2009 US 229746 P; 30.07.2009 US 229747 P; 30.07.2009 US 229748 P; 30.07.2009 US 229751 P; 30.07.2009 US 229752 P; 30.07.2009 US 229755 P; 30.07.2009 US 229761 P; 30.07.2009 US 229767 P; 30.07.2009 US 229778 P; 30.07.2009 US 229786 P; 30.07.2009 US 229789 P; 30.07.2009 US 229796 P; 30.07.2009 US 229735 P
(43) Date of publication of application: 16.05.2012
(73) Proprietor: Kirk Promotion LTD., 114 79 Stockholm (SE)
(72) Inventor: FORSELL, Peter, CH-6300 Zug (CH)
(86) International application number: PCT/SE2010/050833
(87) International publication number: WO 2011/005214

(56) References cited:
- EP-A1- 1 421 919
- EP-A1- 1 421 919
- DE-A1-102004 008 138
- US-A- 4 846 840
- US-A- 4 950 299
- US-A- 6 010 535
- US-A- 6 010 535
- US-A1- 2008 228 283

## Description

### FIELD OF INVENTION

The present invention relates generally to a medical device for implantation in a hipjoint, and a method of providing said medical device.

### BACKGROUND

The hipjoint is a synovial joint, joining the pelvis to the proximal portion of the femoral bone. Synovial joints are the most common types of joints in mammals, and are typical of nearly all limb joints. The contacting surfaces of said the pelvic, the acetabulum, and the contacting surface of the femoral bone, the caput femur, are smooth and rounded, and covered by articular cartilage. A synovial membrane, encapsulates the joint, forming a hipjoint cavity, which contains synovial fluid. Outside the synovial membrane is a fibrous capsule and ligaments, forming an articular capsule.

There are both natural and pathological processes leading to deterioratedjoint function. With age and wear, the articular cartilage becomes less effective as a shock absorber and a lubricated surface. Different degenerative joint diseases, such as arthritis, osteoartrithis, or osteoarthrosis, accelerate the deterioration.

Hipjoint Osteoarthritis is a syndrome in which low-grade inflammation results in pain in the hip joints, caused by abnormal wearing of the Cartilage that acts as a cushion inside if the hipjoint. This abnormal wearing of the cartilage also results in a decrease of the joints lubricating fluid called Synovial fluid. Hipjoint Osteoarthritis is estimated to affect 80% of all people over 65 years of age, in more or less serious forms.

The present treatment for hip osteoarthritis comprises NSAID drugs, local injections of Hyaluronic acid or Glucocorticoid to help lubricating the hip joint, and replacing parts of the hip joint with a prosthesis through hip joint surgery.

The replacing of parts of the hip joint is one of the most common surgeries to date performed at hundreds of thousands of patients in the world every year. The most common method comprises placing a metal prosthesis in Femur and a plastic bowl in Acetabulum. This operation is usually done through a lateral incision In the hip and upper thigh and through, Fascia Lata and the lateral muscles of the thigh. To get access to the hip joint, the supporting hip joint capsule attached to Femur and ilium of Pelvis needs to be penetrated, making it difficult to get a fully functional joint after the surgery. Femur is then cut at the neck with a bone saw and the prosthesis is placed in femur either with bone cement or without. Acetabulum is slightly enlarged using an Acetabular reamer, and the plastic bowl is positioned using screws or bone cement. The complications after hip joint surgery include dislocation of the hip joint and loosening of the prosthesis from its fixation in the femoral bone. The loosening and/or dislocation of the prosthesis could be induced by an abnormal strain being placed on the hip joint from e.g. the patient falling or making a rapid movement of the hip, or by a bodily macrophage reaction.

The surgery typically requires one week of hospitalization due to the Increased risk of infection. The recovery process is on average about 6 weeks, but even after this period the patient should not perform any physical activates that places large strain on the Joint. US6010535 (to Shah) discloses a prosthetic joint having a spherical portion connected to the pelvic bone and adapted to create a functional hip joint by articulating with a bowl shaped surface placed in the caput femur.

### SUMMARY

According to one embodiment of a medical device for implantation in a hip joint of a patient, the natural hip joint having a ball shaped caput femur integrated with a collum femur having a collum and caput center axis, extending longitudinal along the collum and caput femur, in the center thereof, as the proximal part of the femoral bone with a convex hip joint surface towards the centre of the hip joint and a bowl shaped acetabulum as part of the pelvic bone with a concave hip joint surface towards the centre of the hip joint, the medical device comprising; an artificial caput femur comprising a convex surface towards the centre of the hip joint, the convex surface having a center point adapted to be placed, when implanted, on the collum and caput center axis, and an elongated portion adapted to be connected to a prosthetic spherical portion of said artificial convex caput femur and to be fixated inside of a bowl shaped acetabulum as part of the pelvic bone of the human patient, said artificial convex caput femur is adapted to, when implanted, be fixated to the pelvic bone of the human patient through said elongated portion fixation to the pelvic bone, and be in movable connection with a prosthetic artificial acetabulum surface fixated to the femoral bone of the patient, comprising at least one extending portion adapted to clasp said prosthetic spherical portion, wherein said elongated portion comprises a restricting portion adapted to restrict the motion range of the artificial acetabulum in relation to said artificial convex caput femur, and wherein a larger portion of said restricting portion is placed, when implanted, dorsal to a vertical acetabulum plane (PX'Y') coinciding with the collum and caput center axis to enable an advantageous motion range in relation to said artificial acetabulum.

According to one embodiment, said elongated portion comprises at least one recess adapted to receive a portion of said prosthetic artificial acetabulum, when implanted.

According to one embodiment, said recess is adapted to be placed frontal to the coronal pelvis plane, when implanted.

According to one embodiment, said recess is adapted to be placed in the frontal quadrant, when implanted.

According to one embodiment, said recess is adapted to be placed in the dorsal quadrant, when implanted.

According to one embodiment, said recess is adapted to be placed in the proximal quadrant, when implanted.

According to one embodiment, said recess is adapted to be placed in the distal quadrant, when implanted.

According to one embodiment, said recesses is more than 2 mm deep.

According to one embodiment, said recesses is more than 4 mm deep.

According to one embodiment, said recesses is more than 6 mm deep.

According to one embodiment, said recesses is more than 8 mm deep.

According to one embodiment, said elongated portion comprises an adaptation adapted to receive said extending portion when implanted.

According to one embodiment, said adaptation comprises a bent portion of said elongated portion.

According to one embodiment, said bent portion is bent such that said restricting portion of said elongated portion mainly is placed frontal to the coronal pelvis plane, when implanted and being in the base position.

According to one embodiment, said bent portion is bent such that said restricting portion of said elongated portion mainly is placed dorsal to the coronal pelvis plane, when implanted and being in the base position.

According to one embodiment, said bent portion is bent such that said restricting portion of said elongated portion mainly is placed in the frontal quadrant, when implanted and being in the base position.

According to one embodiment, said bent portion is bent such that said restricting portion of said elongated portion mainly is placed in the dorsal quadrant, when implanted and being in the base position.

According to one embodiment, said bent portion is bent such that said restricting portion of said elongated portion mainly is placed in the proximal quadrant, when implanted and being in the base position.

According to one embodiment, said bent portion is bent such that said restricting portion of said elongated portion mainly is placed in the distal quadrant, when implanted and being in the base position.

According to one embodiment, a cross-section of said restricting portion of said elongated portion, perpendicular to the caput and collum center axis, comprises a first distance and a second distance, wherein a center point of a line of said first distance intersects a center point of a line of said second distance, and wherein said first distance is shorter than said second distance.According to one embodiment, said first distance is a cross-sectional distance of a narrow portion if said elongated portion, and wherein said second distance is a cross-sectional distance of a wide portion of said elongated member. According to one embodiment, said restricting portion of said elongated portion is adapted to be substantially aligned with said collum center axis and adapted to be eccentrically placed in relation to said collum axis, when implanted. According to one embodiment, a major portion of said restricting portion of said elongated portion is adapted to be placed frontal to the coronal pelvis plane when implanted.

According to one embodiment, the restricting portion of the elongated portion is adapted to be placed in the frontal quadrant, when implanted.

According to one embodiment, the restricting portion of the elongated portion is adapted to be placed in the dorsal quadrant, when implanted.

According to one embodiment, the restricting portion of the elongated portion is adapted to be placed in the proximal quadrant, when implanted.

According to one embodiment, the restricting portion of the elongated portion is adapted to be placed in the distal quadrant, when implanted.

According to one embodiment, a cross section of the restricting portion of the elongated portion, perpendicular to the collum center axis, is circular.

According to one embodiment, a cross section of the restricting portion of the elongated portion, perpendicular to the collum center axis, is polygonal.

According to one embodiment, a cross section of the restricting portion of the elongated portion, perpendicular to the collum center axis, is elliptical.

According to one embodiment, said restricting portion, when implanted, is adapted to be placed such that adduction is restricted more degrees than flexion.

According to one embodiment, said restricting portion, when implanted, is adapted to be placed such that abduction is restricted more degrees than flexion.

According to one embodiment, said restricting portion, when implanted, is adapted to be placed such that adduction is restricted more degrees than extension.

According to one embodiment, said restricting portion, when implanted, is adapted to be placed such that abduction is restricted more degrees than extension.

According to one embodiment, a medical device system comprises a medical device according to any one of claims 1 - 34 and a prosthetic replacement for the acetabulum comprises at least one extending portion adapted to clasp said spherical portion.

According to one embodiment, said prosthetic replacement for the acetabulum comprises an inner and an outer surface, wherein a contacting portion, of said inner surface is spherical and adapted to face the center of the hip joint when said medical device is implanted, and wherein said prosthetic replacement for the acetabulum is adapted to receive said spherical portion, wherein said prosthetic replacement for the acetabulum comprises at least one extending portion, extending said contacting portion of said inner surface such that said at least one extending portion clasps said spherical portion, such that said spherical portion is restrained in said medical device, and wherein said prosthetic replacement for the acetabulum is adapted to be fixated to the femoral bone of the patient.

According to one embodiment, said prosthetic replacement for the acetabulum is adapted to receive said spherical portion being in connection with said medical device, wherein:
said inner surface comprises an equator line, being the largest circular circumference of said inner contacting surface, being a surface adapted to be in contact with said spherical portion, and
said at least one extending portion passes beyond said equator line, such that the end portion of said contacting portion of said inner surface forms a circular extension line having a smaller circumference than said equator line, and
said at least one extending portion circumferentially extends discontinuously along said equator line, such that a portion of said medical device can be placed between said extension line and said equator line.
According to one embodiment, said extension line is placed proximal to the equator line, when the medical device is implanted.

According to one embodiment, said at least one extending portion extends circumferentially along said equator line, dorsal to the right-left axis of pelvis when being in the base position.

According to one embodiment, said at least one extending portion extends circumferentially along said equator line, dorsal to the coronal pelvis plane PXY and proximal to the horizontal pelvis PXZ plane when being in the base position.

According to one embodiment, said at least one extending portion extends circumferentially along said equator line, dorsal to the coronal pelvis plane PXY and distal to the horizontal pelvis PXZ plane when being in the base position.

According to one embodiment, at least one first extending portion extends circumferentially along said equator line dorsal to the coronal pelvis plane PXY and proximal to the horizontal pelvis PXZ plane, and at least one second extending portion extends dorsal to the coronal pelvis plane PXY and distal to the horizontal pelvis PXZ plane when being in the base position.

According to one embodiment, said at least one extending portion extends circumferentially along said equator line, in the proximal quadrant of the equator line when being in the base position.

According to one embodiment, said at least one extending portion extends circumferentially along said equator line, in the distal quadrant of the equator line when being in the base position.

According to one embodiment, two extending portions extends circumferentially along said equator line, in the distal and proximal quadrant thereof when being in the base position.

According to one embodiment, said at least one extending portion extends circumferentially along said equator line, in the proximal and dorsal quadrant thereof when being in the base position.

According to one embodiment, said at least one extending portion extends circumferentially along said equator line, in the distal and dorsal quadrant thereof when being in the base position.

According to one embodiment, at least one extending portion extends circumferentially along said equator line, in the distal, dorsal and proximal quadrant thereof when being in the base position.

According to one embodiment, at least a first portion of said prosthetic replacement for the acetabulum is an extending portion, extending beyond said circular equator line, and at least a second portion is a portion not extending beyond said circular equator line, wherein said second portion circumferentially extends along at least ¼ of said circular equator line.

According to one embodiment, at least a first portion of said prosthetic replacement for the acetabulum is an extending portion, extending beyond said circular equator line, and at least a second portion is a portion not extending beyond said circular equator line, wherein said second portion circumferentially extends along at least 1 /3 of said circular equator line.

According to one embodiment, at least a first portion of said prosthetic replacement for the acetabulum is an extending portion, extending beyond said circular equator line, and at least a second portion is a portion not extending beyond said circular equator line, wherein said second portion circumferentially extends along at least ½ of said circular equator line.

According to one embodiment, at least a first portion of said prosthetic replacement for the acetabulum is an extending portion, extending beyond said circular equator line, and at least a second portion is a portion not extending beyond said circular equator line, wherein said first portion circumferentially extends along at least ¼ of said circular equator line.

According to one embodiment, at least a first portion of said prosthetic replacement for the acetabulum is an extending portion, extending beyond said circular equator line, and at least a second portion is a portion not extending beyond said circular equator line, wherein said first portion circumferentially extends along at least 1 /3 of said circular equator line.

According to one embodiment; at least a first portion of said prosthetic replacement for the acetabulum is an extending portion, extending beyond said circular equator line, and at least a second portion is a portion not extending beyond said circular equator line, wherein said first portion circumferentially extends along at least ½ of said circular equator line.

According to one embodiment, at least a first portion of said prosthetic replacement for the acetabulum is an extending portion, extending beyond said circular equator line, and at least a second portion is a portion not extending beyond said circular equator line, wherein said first portion circumferentially extends along at least 1/10 of said circular equator line.

According to one embodiment, at least a first portion of said prosthetic replacement for the acetabulum is an extending portion, extending beyond said circular equator line, and at least a second portion is a portion not extending beyond said circular equator line, wherein said first portion circumferentially extends along at least 1/10 of said circular equator line, and said second portion circumferentially extends along at least ¼ of said circular equator line.

According to one embodiment, at least two first portions of said prosthetic replacement for the acetabulum are extending portions, extending beyond said circular equator line, and at least a second portion is a portion not extending beyond said circular equator line, wherein said first portions each circumferentially extends along at least 1/10 of said circular equator line, and said second portion circumferentially extends along at least ¼ of said circular equator line.

According to one embodiment, at least two first portions of said medical device are extending portions, extending beyond said circular equator line, and wherein one of said extending portions extends further than the other extending portion.

According to one embodiment, said medical device further comprises two second portions not extending beyond said circular equator line, and wherein said two first extending portion circumferentially extends along said equator line between said two second portions.

According to one embodiment, said extending portion adapted to clasp said spherical portion and said restricting portion of said elongated portion of said medical device is adapted to, when implanted in said base position, be placed such that their placement in relation to each other creates an advantageous motion range.

According to one embodiment, said extending portion said restricting portion of said elongated portion is adapted to be placed in relation to each other when implanted in said base position, such that adduction is restricted more degrees than flexion.

According to one embodiment, said extending portion said restricting portion of said elongated portion is adapted to be placed in relation to each other when implanted in said base position, such that abduction is restricted more degrees than flexion.

According to one embodiment, said extending portion said restricting portion of said elongated portion is adapted to be placed in relation to each other when implanted in said base position, such that adduction is restricted more degrees than extension.

According to one embodiment, said extending portion said restricting portion of said elongated portion is adapted to be placed in relation to each other when implanted in said base position, such that abduction is restricted more degrees than extension.

The artificial hip joint surface, according to any of the embodiments above may comprise an artificial convex caput femur or an artificial convex caput femur surface or an artificial concave acetabulum or an artificial concave acetabulum surface.

### BRIEF DESCRIPTION OF DRAWINGS

The invention is now described, by way of example, with reference to the accompanying drawings, in which exemplary embodiments the invention as claimed are shown in figs. 21-22.
Fig. 1 a shows pelvis in a frontal view,
Fig. 1b shows pelvis in a frontal view,
Fig. 1c shows the hip joint in section,
Fig. 2a shows pelvis in a lateral view,
Fig. 2b shows pelvis in a lateral view,
Fig. 2c shows pelvis in a perspective view from below,
Fig. 2d shows pelvis in a perspective view from below,
Fig. 2e shows the acetabulum, schematically,
Fig. 2f shows the acetabulum, schematically,
Fig. 2g shows pelvis in a perspective view from below,
Fig. 3a shows the hipjoint in section,
Fig. 3b shows the step of removing a proximal part of the caput femur,
Fig. 4a shows the reaming of the collum and caput femur,
Fig. 4b shows the step of applying an adhesive to the created hemi-spherical cavity in the femoral bone,
Fig. 5 shows the collum and caput femur when a medical device gas been fixated,
Fig. 6a shows the femoral bone when a medical device having a concave contacting surface has been provided to the hemi-spherical cavity,
Fig. 6b shows the femoral bone when a medical device having a concave contacting surface has been provided to the hemi-spherical cavity,
Fig. 6c shows the medical device fixated to the surgically cut caput femur using screws,
Fig. 6d shows the medical device fixated to the femoral bone using fixating portions,
Fig. 6e shows the medical device in which fixating portions are additionally fixated using screws,
Fig. 7a shows the medical device in which the fixating portions extends beyond the greatest circumference of the surgically cut caput femur,
Fig. 7b shows the medical device additionally fixated using a fixating band,
Fig. 7c shows three different embodiment of medical devices comprising fixating portions which are slightly tilted towards the collum and caput femur,
Fig. 7d shows two embodiments in which the concave contacting surface only comprises the part placed inside of the concave cavity,
Fig. 7e shows two embodiments in which the artificial acetabulum surface extends into a portion placed on the surface of a section created when the caput femur is surgically cut,
Fig. 7f describes the medical device fixated using an elongated member, fixating portions, and screws,
Fig. 8 shows the artificial acetabulum surface in further detail,
Fig. 9 shows a section of the medical device according to the embodiment also described with reference to fig. 7a,
Fig. 10a shows the step of milling the periphery of the cortical bone of the caput femur,
Fig. 10b shows the milling of the inside of the cortical bone of the caput femur,
Fig. 11 shows an artificial convex caput femur surface adapted to be placed in an artificial acetabulum surface,
Fig. 12 shows the artificial convex caput femur surface as disclosed with reference to fig. 11 when mounted in an artificial acetabulum surface,
Fig. 13 shows the artificial convex caput femur surface as disclosed with reference to fig. 11 when mounted in an artificial acetabulum surface,
Fig. 14 shows the injection of an adhesive in the acetabulum in the pelvic bone,
Fig. 15 shows the placing of a medical device in the reamed acetabulum surface of the pelvic bone,
Fig. 16a shows the hip joint in section, separated,
Fig. 16b shows the hip joint in section, with a prosthetic acetabulum surface placed in the hemi-spherical cavity in the femoral bone,
Fig. 16c shows the hipjoint in section, with a prosthetic acetabulum surface placed in the hemi-spherical cavity in the femoral bone,
Fig. 16d shows the hipjoint in section, with a prosthetic acetabulum surface placed in the hemi-spherical cavity in the femoral bone,
Fig. 17a - 17d shows embodiments of the medical device, in side views,
Fig. 18 shows the medical device in section, according to one embodiment,
Fig. 19a - 19k shows cross-sections of embodiments of the medical device,
Fig. 20a - 20d shows embodiments of the medical device in sectional side view and in cross-section.
Fig. 21 a shows pelvis in a lateral view,
Fig. 21b shows the hip joint in section,
Fig. 22a - 22c shows cross-sections of the medical device,
Fig. 23 shows a prosthetic replacement for the acetabulum, according to one embodiment,
Fig. 24 shows the hipjoint in section, when a prosthetic replacement for the acetabulum, and a medical device has been implanted,
Fig. 25a shows the pelvic region in a frontal view,
Fig. 25b shows the pelvic region in a frontal view,
Fig. 26 shows the pelvic region in a frontal view,
Fig. 27 shows a medical device placed in the femoral bone,
Fig. 28a shows the femoral bone in a lateral view,
Fig. 28b shows the femoral bone in a lateral view,
Fig. 29 shows the femoral bone in a lateral view,
Fig. 30 shows the femoral bone in a lateral view,
Fig. 31 shows the femoral bone in a lateral view,
Fig. 32 shows a medical device placed in the femoral bone,
Fig. 33a - 33f shows embodiments of prosthetic replacements for the acetabulum,
Fig. 34a - 34d shows embodiments of the medical device in combination with embodiments of the prosthetic replacement for the acetabulum, in a sectional side view and in cross-section.

### DETAILED DESCRIPTION

The hipjoint is a synovial ball and socketjoint which permits a large motion range for allowing a plurality of different movements of the lower limb. From a neutral position the following movements of the hipjoint are normally possible: Lateral or external rotation, 30° with the hip extended, 50° with the hip flexed, medial or internal rotation 40°, extension or retroversion 20°, flexion or anteversion 140°, abduction 50° with hip extended, 80° with hip flexed, adduction 30° with hip extended, 20° with hip flexed.

When replacing the natural hip joint with a prosthetic hip joint, the depth of the prosthetic acetabulum will affect the motion range, the deeper the acetabulum bowl is made the more restrictive it is to the motion range. A deeper bowl has the advantage of reducing the risk of hipjoint luxation, the risk of which is a major drawback with prosthetic hips of today.

The anatomy of the hipjoint and its surroundings is further disclosed in: Marieb et al., Human Anatomy, 2003, Benjamin Cummings, San Francisco, pages 195 - 202 and in Moore et al., Clinically oriented anatomy,1999, Lippincott, Williams & Wilkins, Baltimore, pages 501 - 653, both hereby incorporated by reference.

Centrally in the body should herein be understood as a point of reference located at the intersection of the Median plane and the Coronal plane and in the center part of the heart along a longitudinal axis (Caudal - Cranial). Proximal and distal are direction or location terms used in relation to said point centrally in the body and hence a distal point is a point farther away from the central point in relation a proximal point of the same structure. Any plane disclosed herein is to be understood as having infinite extension. Other anatomical terms used herein are further described in Moore et al., Clinically oriented anatomy, 1999, Lippincott, Williams & Wilkins, Baltimore, pages 2 - 10, which is hereby incorporated by reference.

Functional hip movements are to be understood as movements of the hip that at least partly correspond to the natural movements of the hip. On some occasions the natural movements of the hip joint might be somewhat limited or altered after hip joint surgery, which makes the functional hip movements of a hip joint with prosthetic surfaces somewhat different than the functional hip movements of a natural hip joint.

Everyday activities is to be understood as activities which are not connected to any extreme movements, such that some physical sports require. For example, everyday activities comprise: walking, sitting, cycling etc.

The functional position of an implantable medical device or prosthesis is the position in which the hip joint can perform functional hip movements. The final position is to be understood as a functional position in which the medical device needs no further position change to function.

Arthroscopy is to be understood as key hole surgery performed in a joint, since the arthroscopic procedure could be performed in the abdomen of the patient some of the steps of this arthroscopic procedure is more laparoscopic, however for the purpose of this invention the two terms arthroscopy and laparoscopy is used synonymously and for the purpose of this invention the main purpose of these methods are is that they are minimally invasive.

Elastic deformation is when a material deforms under stress (e.g. external forces), but returns to its original shape when the stress is removed. A more elastic material is to be understood as a material having a lower modulus of elasticity. The elastic modulus of an object is defined as the slope of its stress-strain curve in the elastic deformation region. The elastic modulus is calculated as stress / strain, where stress is the force causing the deformation, divided by the area to which the force is applied; and strain is the ratio of the change caused by the stress.

Elasticity is to be understood as a materials ability to deform in an elastic way.

Stiffness is to be understood as the resistance of an elastic body to deformation by an applied force.

Biocompatible material is to be understood as being a material with low level of immune response. Biocompatible materials are sometimes also referred to as biomaterials. Analogous is biocompatible metals a biocompatible metal with low immune response such as titanium or tantalum. The biocompatible metal could also be a biocompatible alloy comprising at least one biocompatible metal.

Form fitting is to be understood as an element having a part or section which is adapted to enable a mechanical connection of said element to at least one other element using said part or section. Form fitted structure is a structure of an element which enables form fitting.

In the following a detailed description of embodiments of the present invention will be given. In the drawing figures, like reference numerals designate identical or corresponding elements throughout the several figures. It will be appreciated that these figures are for illustration only and are not in any way restricting the scope of the invention. Thus, any references to direction, such as "up" or "down", are only referring to the directions shown in the figures. Also, any dimensions etc. shown in the figures are for illustration purposes.

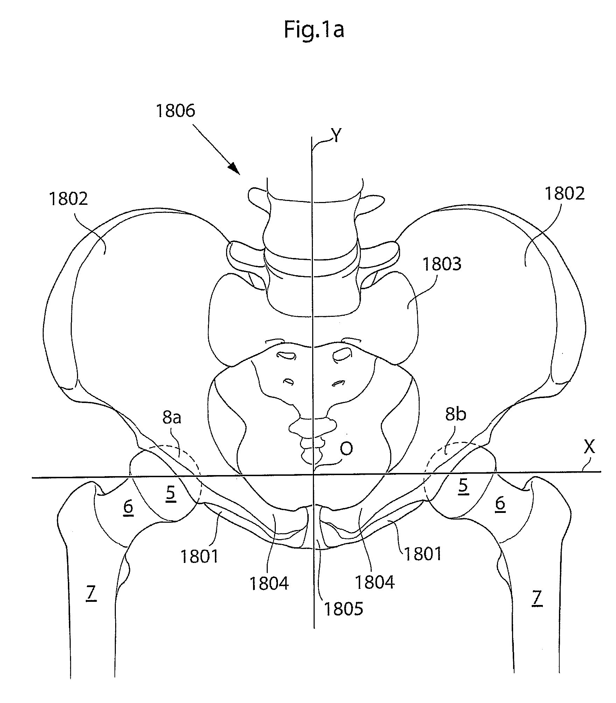

Fig. 1a shows the pelvis in a frontal view. Pelvis comprises the right and left hip bone making up the pelvic bone, in turn comprising the Sacrum 1803, Ilium 1802, Pubis 1804 and Ischium 1801. The hip joint houses the right and left acetabulum 8a,b placed laterally and distally in the pelvis. The acetabulum 8a,b being a spherically shaped cavity in the hip bones making up one of the parts of the hip joint, the acetabulum 8a,b being adapted to house the caput femur 5, being the proximal portion of the femoral bone 7 having a spherical contacting surface adapted to be placed in the acetabulum 8a,b and thus creating the operable hip joint. The pelvis has a right-left axis X extending substantially from the bottom of the left acetabulum 8a to the bottom of the right acetabulum 8b, the pelvis further having a caudal-cranial axis Y extending perpendicular to said right-left axis, centrally and substantially along the length of the patient, passing the dorsal portions of the pubic symphysis 1805 and substantially following the spinal cord 1806, intersecting the left-right axis X.

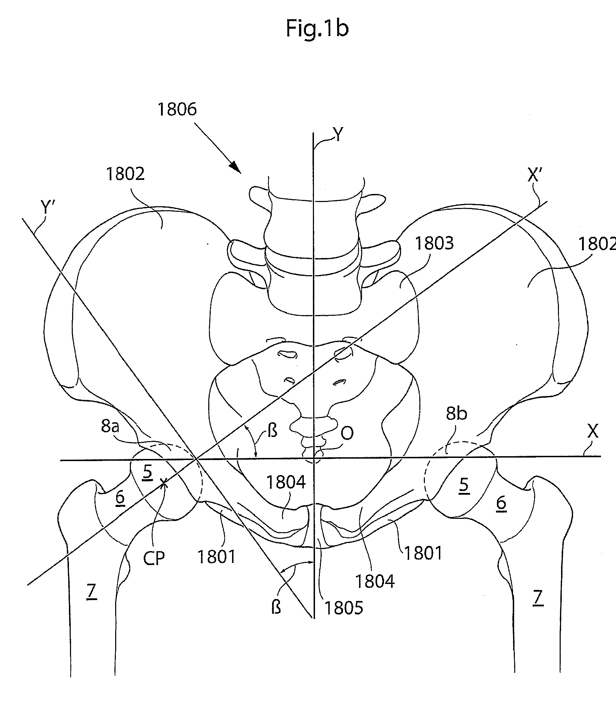

Fig. 1b shows the pelvis in a frontal view disclosing a second, displaced coordinate system. The second displaced coordinate system has its origin O' in the bottom of the acetabulum bowl 8a. The axis X and Y have, in a frontal view, been rotated the angle β, creating the axis X' and Y'. The axis X' being aligned with the caput and collum femur center axis CX, when the hip joint is in its base position when the patient is standing up or lying down. In said base position, the axis X' goes through a point O' being the origin O' in the bottom of the acetabulum bowl 8a, and a center point CP, being a point in the center of a circle defined by the edges of the acetabulum bowl 8a, and further trough the top of the caput femur 5 and following inside of the collum femur 6, aligned with the collum femur 6. The axis Y' is perpendicular to the axis X' and goes through the origin O' in the bottom of the acetabulum bowl 8a, parallel to a plane defined by the circle defined by the edges of the acetabulum bowl 8a.

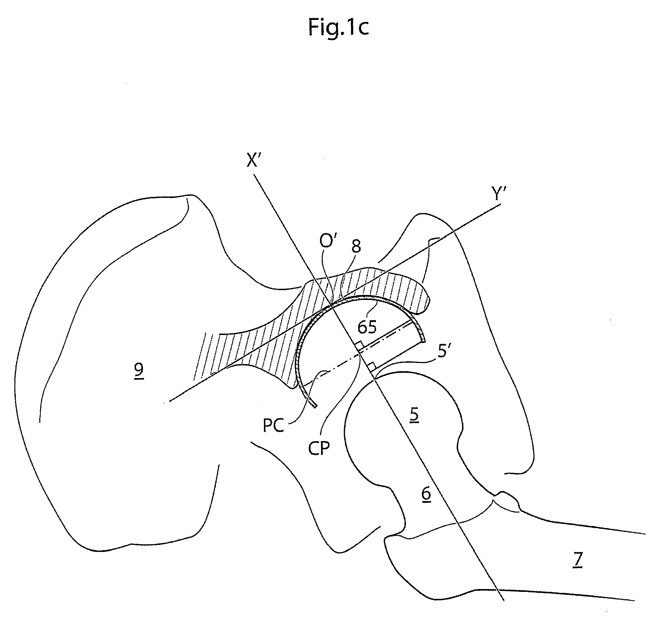

Fig. 1c shows the right pelvic bone 9 in section disclosing the second, displaced coordinate system. The origin O' is in the bottom of the acetabulum bowl 8. The axis X' is aligned with the caput 5 and collum 6 femur center axis CX, when the hip joint is in its base position when the patient is standing up or lying down with extended leg. In said base position the axis X' is goes through a point O' being the bottom of the acetabulum bowl 8, and a center point CP, being a point in the center of a circle defined by the edges of the acetabulum bowl 8, and further trough the top of the caput femur 5' and following inside of the collum femur 6, aligned with the collum femur 6. The axis Y' is perpendicular to the axis X', goes through the origin O' in the bottom of the acetabulum bowl 8, parallel to the plane PC defined by the circle defined by the edges of the acetabulum bowl 8.

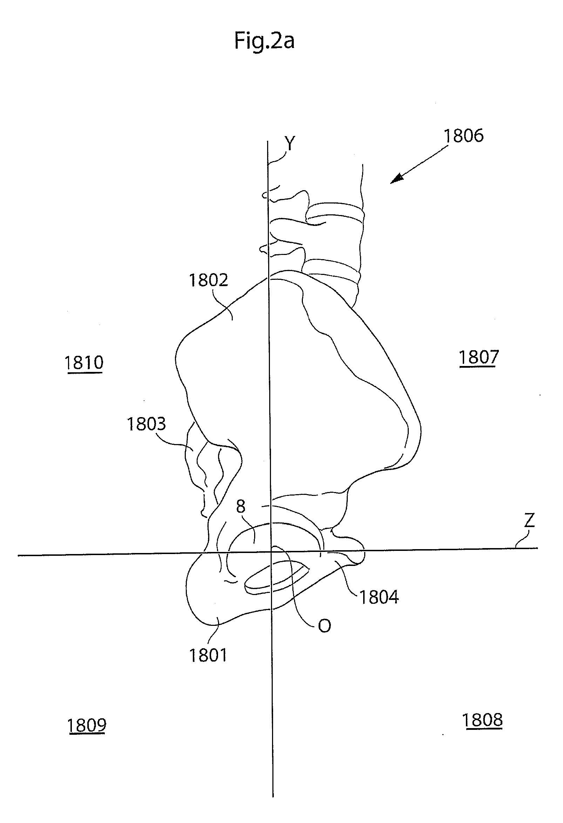

Fig.2a shows the pelvis in a lateral view, thus displaying the posterior side of Ilimu 1802, the anterior side of ichum 1801, the anterior side of Pubis 1804, and Sacrum 1803 in a lateral view. The pelvis has furthermore a dorsoventral axis Z being perpendicular to the caudal-cranial axis Y and the right-left axis X shown in fig. 1, and intersecting them both creating a common origin O for the three axis X,Y,Z. The dorsoventral axis Z and the caudal-cranial axis Y thus being oriented such that a horizontal pelvis plane PXZ extends from the dorsoventral axis Z, and a coronal plane PXY extends from the caudal-cranial axis Y.

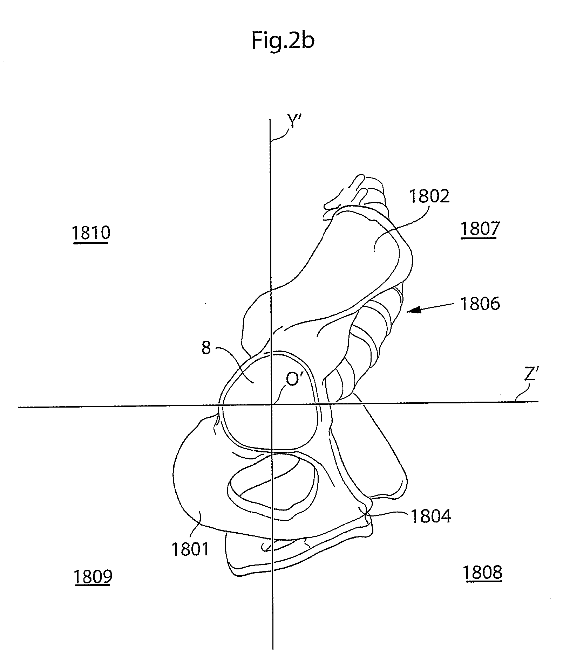

Fig. 2b shows the pelvis in a plane view from the side and slightly from below, in the direction of the axis X' (further disclosed with reference to figs 1b and 1c). The view of fig 2b displaying the axis Y' and Z' with origin O' in the bottom of the acetabulum bowl 8 making up the acetabulum coordinate system. The axis Y', Z', in this plane view, dividing the acetabulum bowl 8 into four quadrants: the proximal-frontal quadrant 1807, the distal-frontal quadrant 1808, the distal-dorsal quadrant 1809 and the proximal-dorsal quadrant 1810.

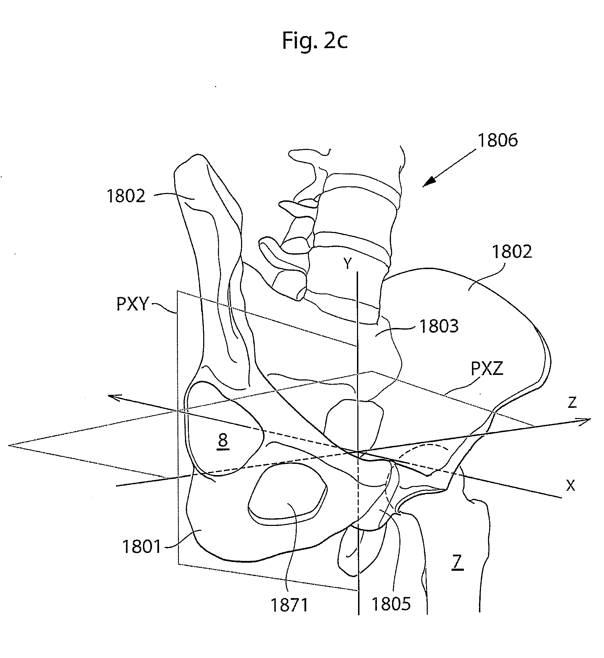

Fig. 2c shows the pelvis in a perspective view from below and slightly from the front, displaying the right-left axis X passing through the center of the right and left acetabulum 8. The right-left axis X is perpendicular to the dorsoventral axis Z which also is perpendicular to the caudal-cranial axis Y. The coronal plane PXY extends from the dorsoventral axis Y, and the horizontal pelvis plane PXZ extends from the dorsoventral axis Z, thus being perpendicular to the coronal plane PXY.

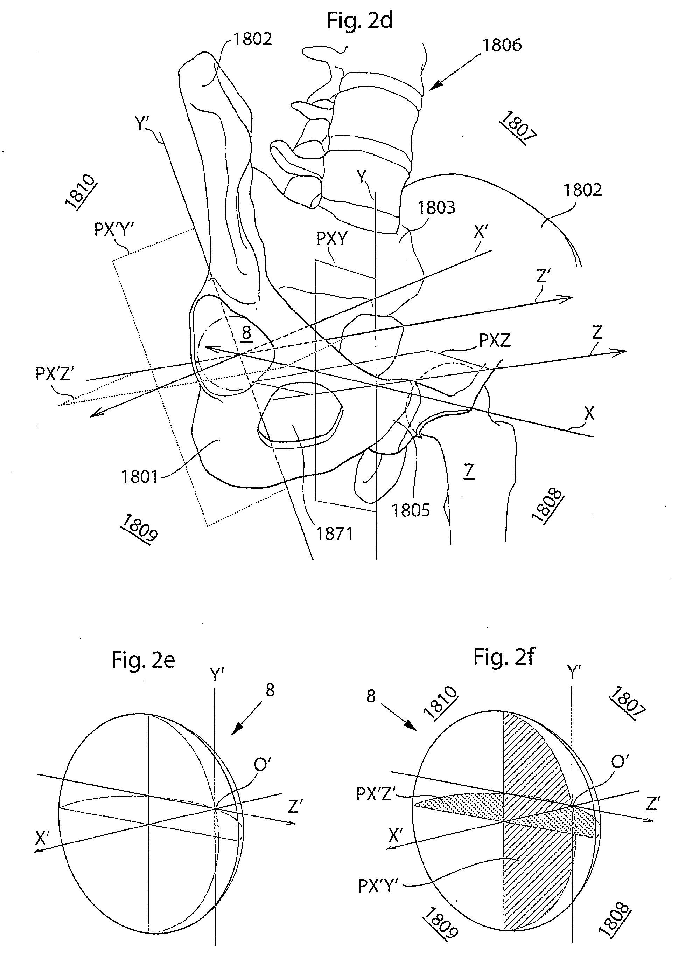

Fig. 2d shows the coordinate system X,Y,Z and planes PXY, PXZ of fig. 2c, and the second, displaced, coordinate system X', Y', Z' being the coordinate system of the acetabulum 8, also shown in fig. 2b. The axis of the coordinate system of the acetabulum X', Y', Z' having their origin O' in the bottom of the acetabulum bowl 8, the axis X' being aligned with the caput and collum center axis. Fig. 2d further discloses the vertical acetabulum plane PX'Y' and the horizontal acetabulum plane PX'Z', PX'Y' being defined by the axis X',Y' and the vertical acetabulum plane PX'Z' being defined by the axis X',Z'. The planes PX'Y' and PX'Z' dividing the acetabulum bowl 8 into four quadrants, the proximal-frontal quadrant 1807, the distal-frontal quadrant 1808, the distal-dorsal quadrant 1809 and the proximal-dorsal quadrant 1810, in accordance with what is previously disclosed, with reference to fig. 2b. Fig. 2d further shows the location of foramen obturatum 1871.

Fig. 2e shows, schematically how the acetabulum coordinate system X',Y',Z' relates to the hemisphere defined by the acetabulum bowl 8.

Fig. 2f shows, schematically, how the vertical acetabulum plane PX'Y', and the horizontal acetabulum plane PX'Z' divides the acetabulum 8 into four quadrants; the proximal-frontal quadrant 1807, the distal-frontal quadrant 1808, the distal-dorsal quadrant 1809 and the proximal-dorsal quadrant 1810, in accordance with the previously disclosed, with reference to fig. 2b and 2d.

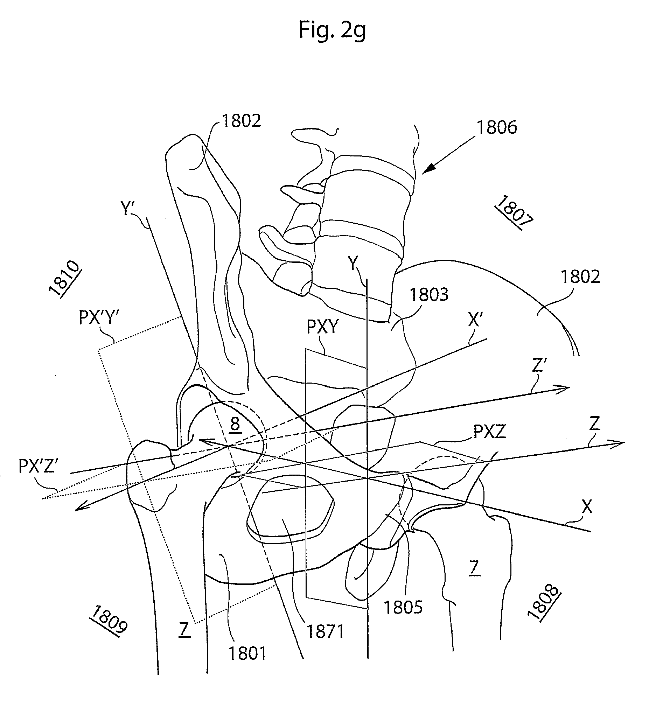

Fig 2g shows the view of fig. 2d, and in addition it shows the horizontal and vertical acetabulum planes PX'Y' and PX'Z' also being the caput and collum femur horizontal and vertical planes PX'Y' and PX'Z', analogically dividing the caput and collum femur into four quadrants.

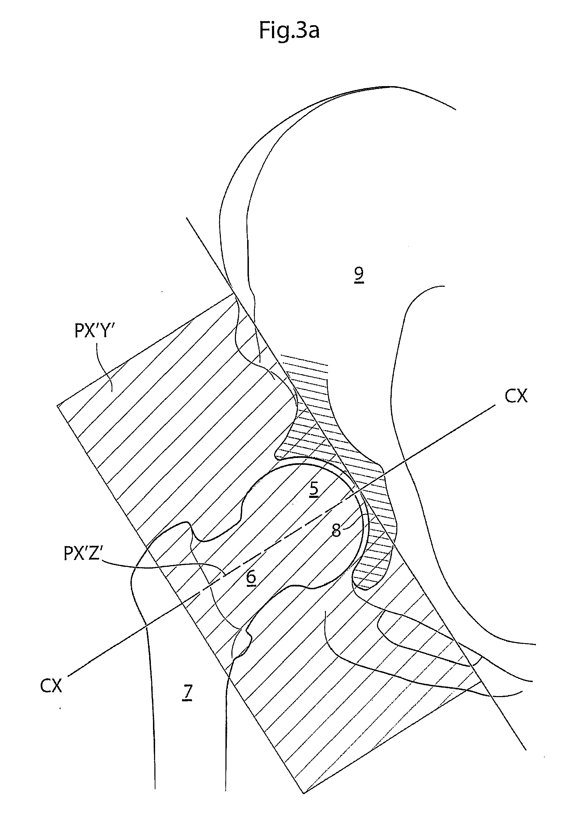

Fig. 3a shows a perspective view of the hip joint in section, displaying the horizontal and vertical planes PX'Y' and PX'Z' originating from the caput 5 and collum 6 center axis CX and dividing the caput 5 and collum 6 femur into quadrants.

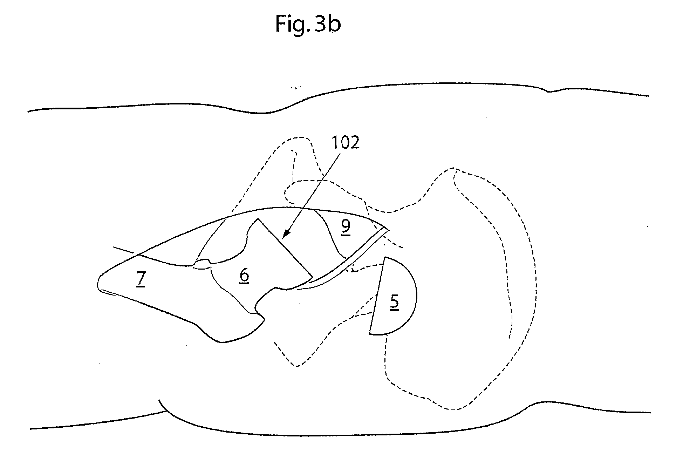

Fig. 3b shows the proximal part of the caput femur 5 being removed e.g. by means of a bone saw. A surface of a section 102 is thus created perpendicularly to a length axis of the collum femur 6

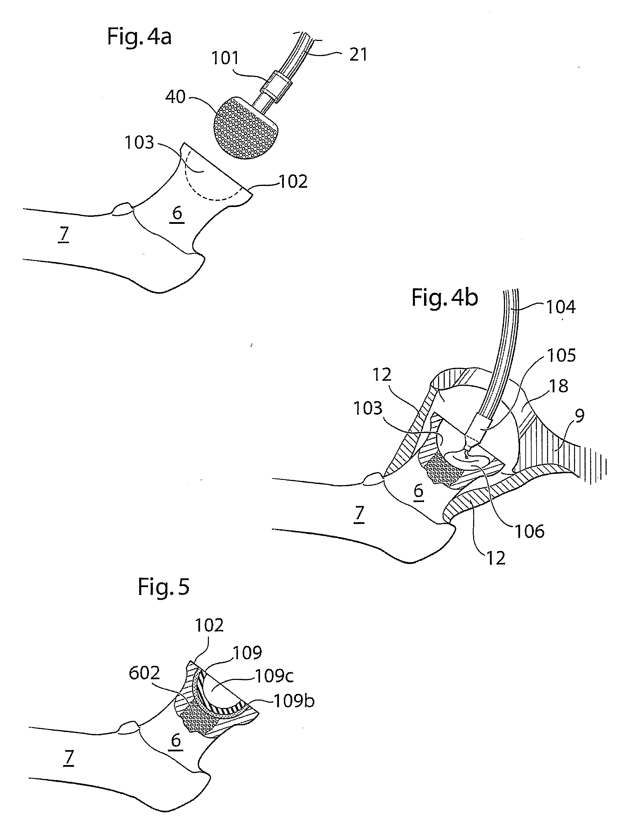

Fig. 4a shows the reaming of the collum femur 6 and caput femur 5 using a reamer 40 connecting to an elongated member 21 by a connecting section 101. The reamer 40 creating a hemi-spherical cavity, having a concave surface 103, centrally placed in the caput 5 and collum femur 6.

Fig. 4b shows the step of applying an adhesive 106 to the created hemi-spherical cavity in the femoral bone using an injecting member 104 having an injecting nozzle 105. In the embodiment shown in fig. 4b the injecting member is inserted into an area of the hip joint through a hole 18 in the pelvic bone 9, however it is equally conceivable that the injecting member is inserted through the hip joint capsule 12 or the femoral bone 7.

Fig. 5 shows the femoral bone 7 when a medical device 109 having a concave contacting surface has been provided to the hemi-spherical cavity, centrally placed in the caput 5 and collum femur. An elastic layer 109b adapted to absorb shocks from the femoral bone has been placed between the surface 109c adapted to be in contact with the artificial caput femur surface, and the femoral bone 7, 6. The elastic layer 109b could be an elastic polymer layer; such as a polyurethane or silicone layer. Having a layer absorbing shocks in the hip joint reduces the risk of fastening elements in contact with bone being affected by strains such that the fastening elements are loosened from their respective fastening positions, it also increases the comfort for the patient.

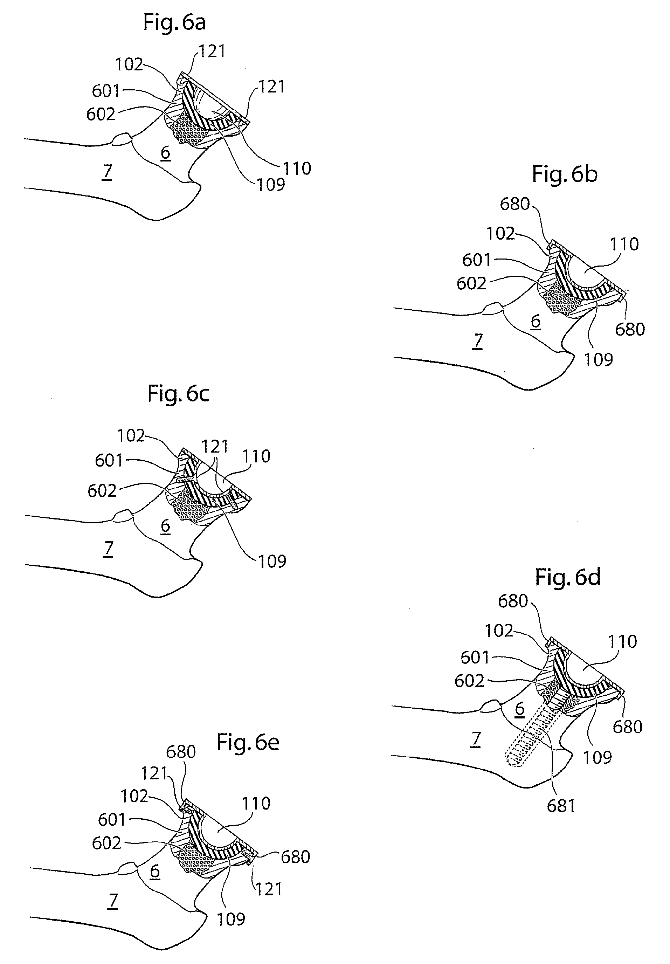

Fig. 6a shows the femoral bone 7 when a medical device having a concave contacting surface 110 has been provided to the hemi-spherical cavity, centrally placed in the caput 5 and collum femur. The medical device has been fixated to the femoral bone 7 using screws 121 placed aligned with the caput and collum femur center axis and entering the cortical bone of the caput femur.

Fig. 6b shows the femoral bone 7 when a medical device having a concave contacting surface 110 has been provided to the hemi-spherical cavity, centrally placed in the caput 5 and collum femur. The medical device comprises fixating portions 680 extending on the outside of the surface of a section 102 of the surgically cut caput femur, comprising cortical bone in the periphery thereof, thereby stabilizing the medical device with the artificial concave acetabulum surface 110 in the surgically cut caput femur.

Fig. 6c shows an alternative embodiment, in which the medical device has been fixated to the surgically cut caput femur using screws 121 entering the cortical bone 601 of the caput femur.

Fig. 6d shows yet another embodiment, in which the medical device is fixated to the femoral bone using fixating portions, in accordance with the embodiment described with reference to fig. 6b, and an elongated member 681. The elongated member is according to this embodiment a threaded member 681 extending along the collum and caput femur center axis, in the cancellous bone 602 of the collum femur, and entering the cortical bone 601 of the femoral bone, on the inside thereof, in the area of the greater trochanter. The threaded elongated member 681 creates an axial force when pulled pressing the medical device towards the surface of a section 102 of the surgically cut caput femur, thereby stabilizing and fixating the medical device in the concave cavity created in the caput femur.

Fig. 6e shows yet an alternative embodiment of the medical device in which the fixating portions 680 are additionally fixated using screws 121 placed from the outside of the surgically cut caput femur, perpendicularly to the collum and caput femur center axis.

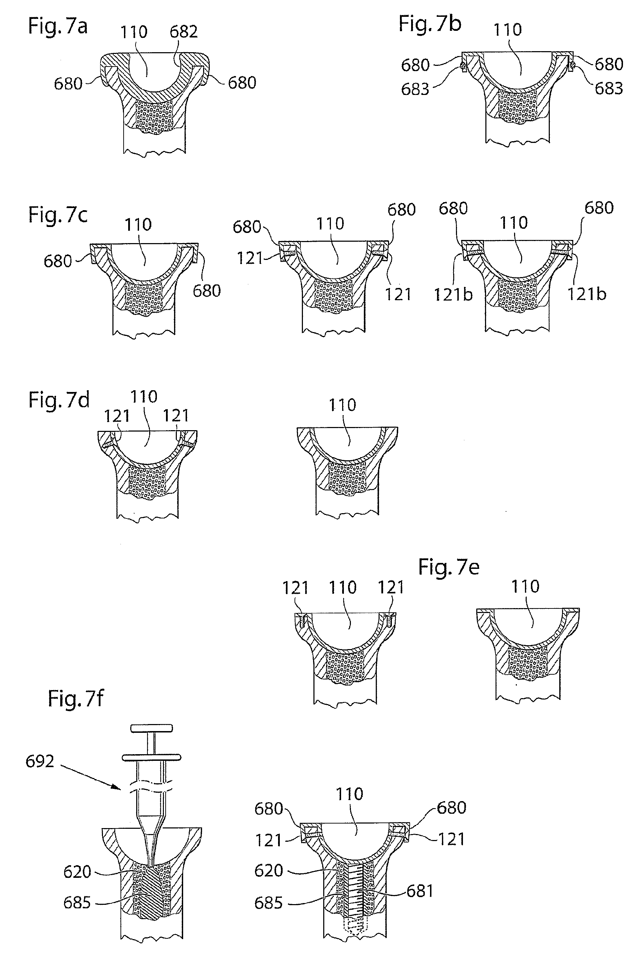

Fig. 7a shows the medical device in an embodiment in which the fixating portions 680 extends beyond the greatest circumference of the surgically cut caput femur and thereby clasps the medical device to the surgically cut caput femur, fixating the medical device thereon. The concave contacting surface 110 is also adapted to travel beyond the equator of an artificial caput femur which is placed in the artificial acetabulum when mounted into a functioning artificial hip joint, and clasping the artificial caput femur when mounted therein.

Fig. 7b shows yet another embodiment where the medical device is additionally fixated using a fixating band 683 encircling the fixating portions of the medical device and thereby further clasping the medical device to the surgically cut caput femur.

Fig. 7c shows three different embodiment of medical devices comprising fixating portions 680 which are slightly tilted towards the collum and caput femur center axis, thereby clasping a portion of the surgically cut caput femur for fixating the medical device to the surgically cut caput femur. The three different embodiments shown is first, without screws 121, second, with screws entering the cortical bone, and third, with screws penetrating the cortical bone and entering the medical device on the inside of the concave cavity, which enables the screws to squeeze a portion of the cortical bone for tight fixation of the medical device.

Fig. 7d shows two embodiments in which the concave contacting surface 110 only comprises the part placed inside of the concave cavity. The first embodiment shows the acetabulum surface 110 fixated to the concave cavity using screws 121, whereas the second embodiment shows the artificial acetabulum surface fixated without screws, such as using an adhesive.

Fig. 7e shows two embodiments in which the artificial acetabulum surface extends into a portion placed on the surface of a section created when the caput femur is surgically cut. In the first embodiment the medical device is fixated using screws entering the cortical bone, whereas in the second embodiment the artificial contacting surface is fixated without screws, such as using an adhesive.

Fig. 7f describes an embodiment in which the medical device is further fixated using an elongated member 681, fixating portions 680, and screws 121 placed between the fixating portions 680 and the inside of the artificial acetabulum contacting surface 110. The elongated member 681 is according to this embodiment a threaded member 681 and the first fig. discloses the preparation of the cancellous bone 602 with a curing fluid 685, such as bone cement, creating a sturdy base for the fixation of the threaded member 681.

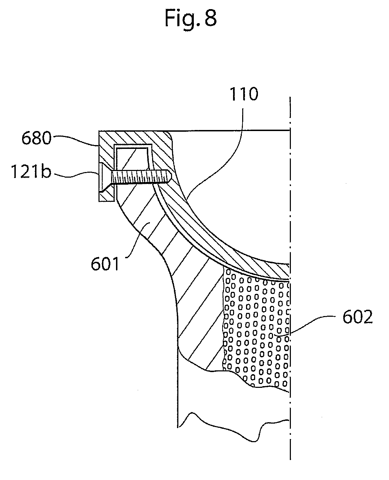

Fig. 8 shows the artificial acetabulum surface 110 in further detail when the artificial acetabulum surface comprises a fixating portion 680 extending on the outside of the cortical bone 601. The fixating portion 680 is further fixated using screws 121 placed from the outside, through a hole in the medical device, penetrating the cortical bone 601 of the surgically cut caput femur and entering the medical device placed in the concave cavity in the caput femur.

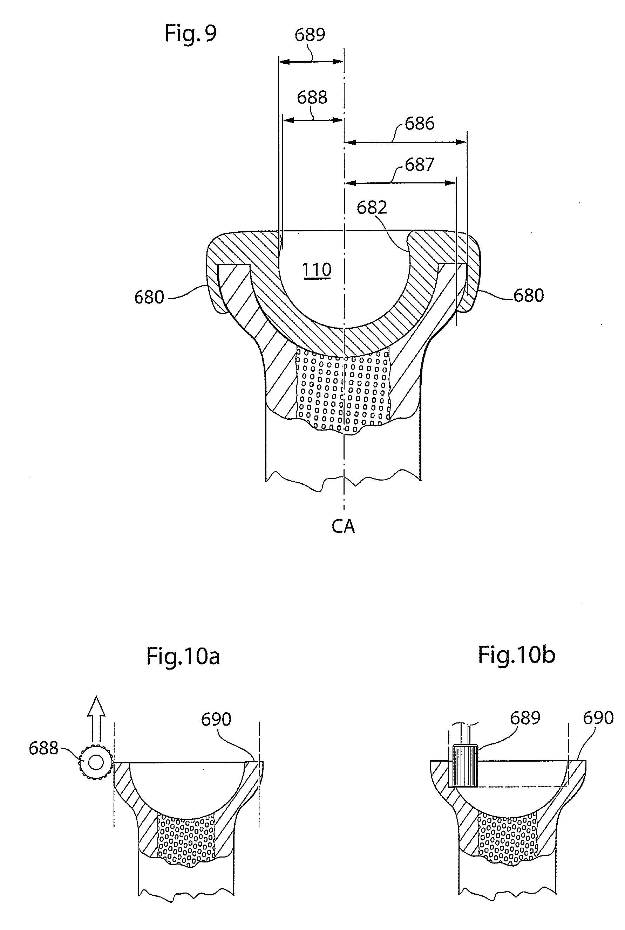

Fig. 9 shows a section of the medical device according to the embodiment also described with reference to fig. 7a, in further detail. The medical device according to the embodiment in fig. 9 comprises fixating portions 680 which reaches on the outside of the surgically cut caput femur and clasps the cortical bone of the caput femur. The medical device clasps the caput femur since a distance 687, between the collum and caput femur center axis CA and the fixating portion in shorter than a distance 686 between the collum and caput femur center axis CA and a portion of the fixating portion placed more proximally when the medical device is implanted. On the inside of the artificial concave acetabulum surface, the surface extends beyond the equator of the artificial caput femur adapted to be placed therein. An extending portion 682 clasps the artificial caput femur placed in the artificial acetabulum surface 110 since a distance 688, between the collum and caput femur center axis CA and the inside of the artificial acetabulum surface 110 is shorter than a distance 689 between the collum and caput femur center axis CA and a point on the inside of the artificial acetabulum contacting surface 110 being more distal when the medical device is implanted.

Fig. 10a shows the step of milling the periphery 690 of the cortical bone of the caput femur after the caput femur has been surgically cut, using a milling device 688 adapted therefor. The milling process creates a straighter edge which facilitates the fixation of a medical device on the outside of the caput femur.

Fig. 10b shows the milling of the inside of the cortical bone of the caput femur after the caput femur has been surgically cut, using a milling device 689 adapted therefore, creating a straighter edge which facilitates the fixation of a medical device on the inside of the caput femur.

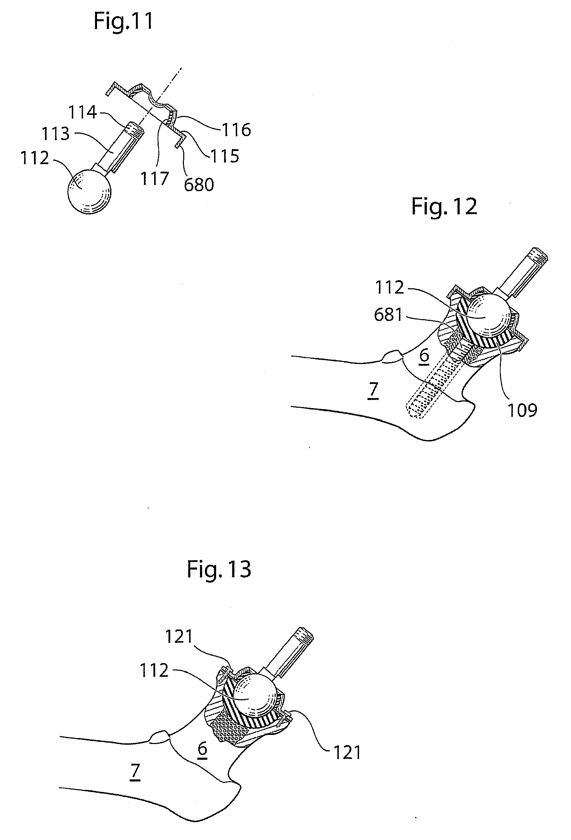

Fig. 11 shows an artificial convex caput femur surface 112 adapted to be placed in an artificial acetabulum surface according to any of the embodiments herein. After the artificial convex caput femur surface has been placed in the artificial acetabulum surface it is locked using a locking member 116 comprising a surface 117 adapted to be in contact with the artificial convex hip joint surface 112. The locking member 116 further comprises fixating members 115 which are adapted to assist in the fixation of the locking member 116 to the caput femur 5 or collum femur 6, which in turns fixates the artificial convex hip joint surface 112. The fixating members comprises a fixating portion 680 which travels on the outside of the surgically cut caput femur for radially stabilizing and fixating the locking member to the surgically cut caput femur. The artificial convex hip joint surface 112 is fixated to an elongated attachment rod 113 comprising a thread 114 and a recess 2203 for enabling further movement of the prosthetic replacement for the acetabulum 110 in relation to the spherical portion 112 connected to elongated portion 2201.

Fig. 12 shows the artificial convex caput femur surface 112 as disclosed with reference to fig. 11 when mounted in an artificial acetabulum surface 109 placed in a concave cavity in the femoral bone. The artificial acetabulum surface is according to this embodiment is fixated to the femoral bone using an elongated member 681, here being a threaded member placed aligned with the collum and caput center axis.

Fig. 13 shows the artificial convex caput femur surface 112 as disclosed with reference to fig. 11 when mounted in an artificial acetabulum surface 109 placed in a concave cavity in the femoral bone. The artificial acetabulum surface is according to this embodiment is fixated using screws 121 entering the cortical bone of the surgically cut caput femur.

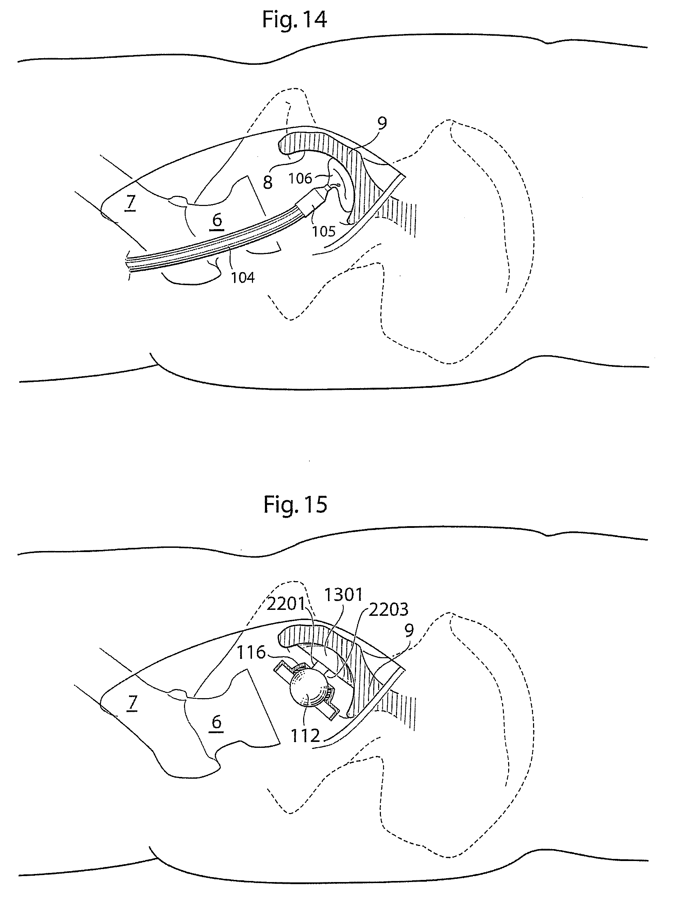

Fig. 14 shows the injection of an adhesive 106 in the acetabulum 8 in the pelvic bone 9 using an injecting member comprising an injecting nozzle 105, which is a preparation for the fixation of a medical device to the pelvic bone 9.

Fig. 15 shows the placing of a medical device in the reamed acetabulum 8 surface of the pelvic bone 9. The medical device comprises a convex hip joint surface 112 fixated to a fixation element 1301, which in turn is fixated to the acetabulum 8 using the injected fluid, which could be assisted or replaced by a mechanical fixation element such as screws. The medical device further comprises a pre-mounted locking member 116 for locking the convex hip joint surface of the concave hip joint surface placed in the caput 5 and collum femur 6 for hindering dislocation of the hip joint when the hipjoint is in its functional position. The artificial convex hipjoint surface 112 is fixated to an elongated attachment rod 113 comprising a thread 114 and a recess 2203 for enabling further movement of the prosthetic replacement for the acetabulum 110 in relation to the spherical portion 112 connected to elongated portion 2201.

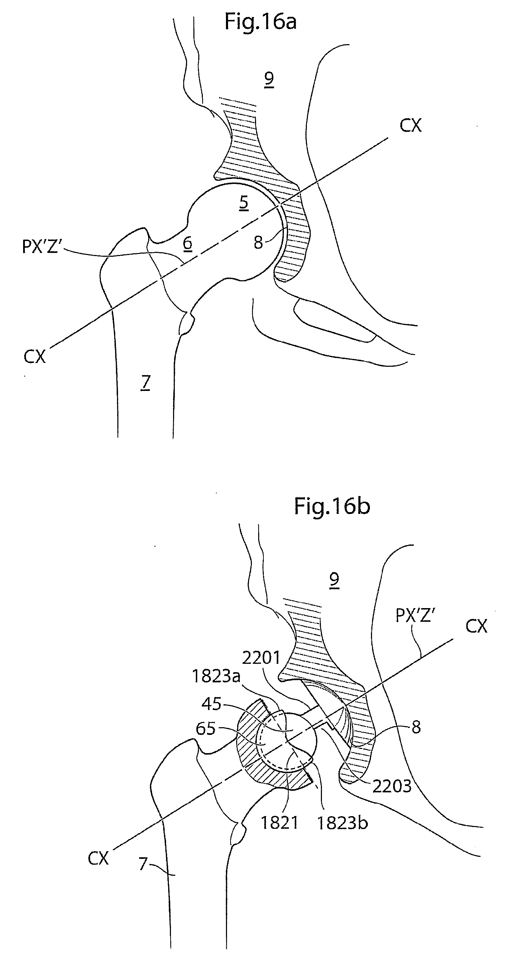

Fig. 16b shows the view of fig. 16a when a prosthetic acetabulum surface 65 has been placed in the hemi-spherical cavity in the femoral bone replacing the natural contacting surface of the acetabulum 8. The prosthetic replacement for the acetabulum 8 comprises two extending portions 1823a,b adapted to clasp the spherical portion 45 being a prosthetic part adapted to replace the natural collum femur 5 and thus further restraining the prosthetic spherical replacement for the caput femur 45 in the prosthetic replacement for the acetabulum 65. The prosthetic replacement for the acetabulum 65 and the prosthetic spherical replacement for the caput femur 45 combined creating a functional prosthetic hipjoint. Fig. 16b further shows an elongated portion 2201, adapted to at least partially replace the collum femur. The prosthetic spherical portion 45 and the elongated portion 2201 are connected to each other at the proximal portion of the elongated portion 2201. According to the embodiment shown in fig. 16b the elongated portion 2201 is adapted to be placed and fixated inside of bowl shaped acetabulum 8 as part of the pelvic bone 9. The elongated portion 2201 restricts the movement of the prosthetic replacement for the acetabulum 65 in relation to the spherical portion 45, especially since the prosthetic replacement for the acetabulum comprises extending portions 1823a,b extending beyond the equator line of the prosthetic replacement for the acetabulum. The elongated portion thus further comprises a recess 2203 for enabling further movement of the prosthetic replacement for the acetabulum 110 in relation to the spherical portion 112 connected to elongated portion 2201.

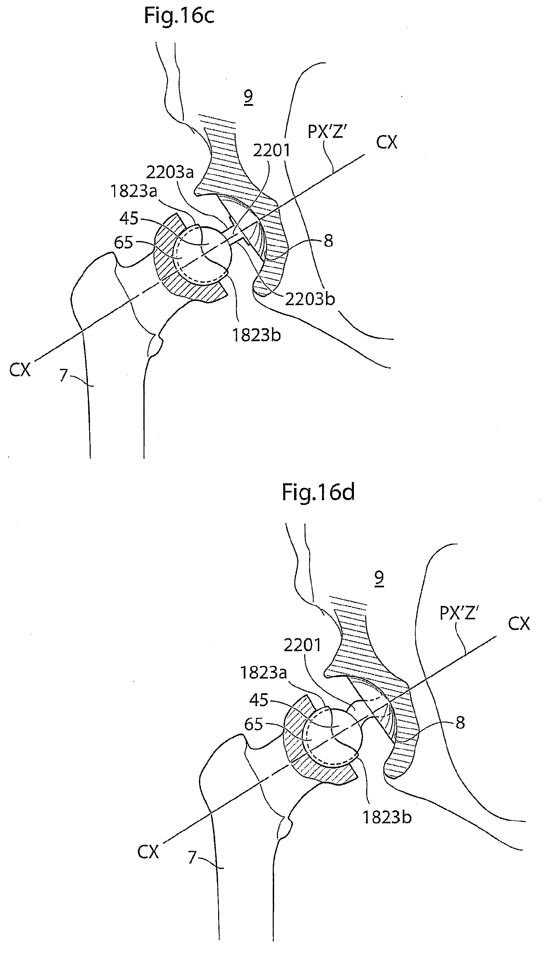

Fig. 16c shows a perspective view of the hip joint in section, similar to the embodiment shown in fig. 16b. However, in the embodiment of fig. 16c the elongated portion 2201 comprises two recesses 2203a,b, placed such that the elongated portion 2201 are adapted to receive the extending portions 1823a,b through the extending portions 1823a,b entering the recesses 2203a,b of the elongated portion 2201 when the prosthetic replacement for the acetabulum 65 move in relation to the elongated 2201 and spherical 45 portions.

Fig. 16d shows a perspective view of the hip joint in section, similar to the embodiment shown in fig. 16b and 16c. However, in the embodiment of fig. 16d the elongated portion 2201 is shaped such that the contacting portion between the elongated portion 2201 and the spherical portion 45 is eccentrically placed in relation to the center axis CX of the collum and caput femur. The eccentrically placed contacting portion enables replacement for the acetabulum to move more in one direction than in another. This embodiment, similar to the recess embodiments previously disclosed, enables the adaption of the elongated member to the prosthetic replacement for the acetabulum 65, and in particular the extending portions 1823a,b thereof, for enabling a particular motion pattern of the hip joint and thus the leg.

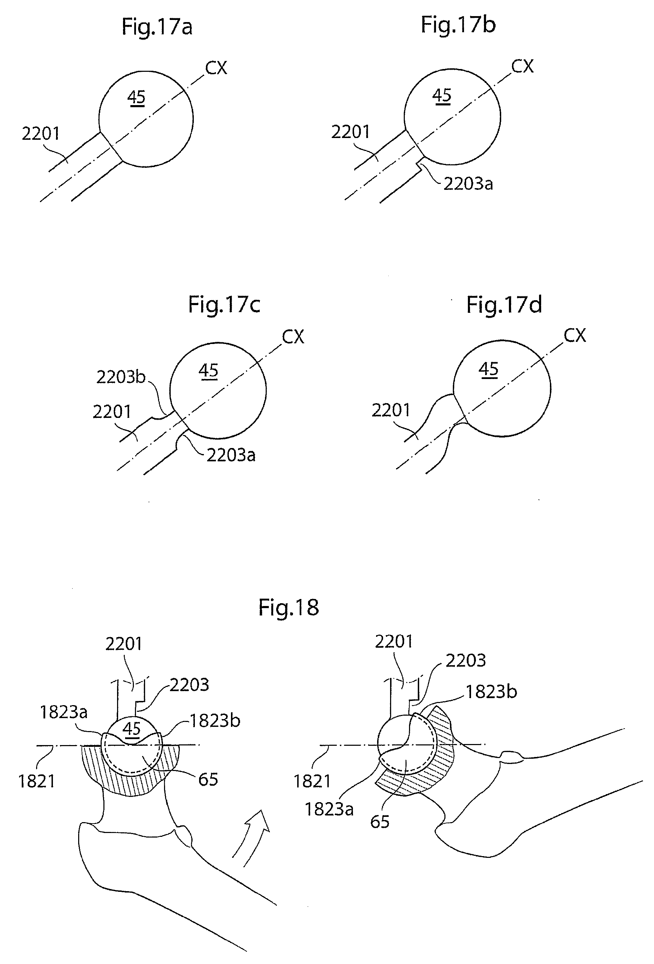

Fig. 17a shows a prosthetic spherical portion 45 and a prosthetic elongated portion 2201 in a schematic view, displaying the prosthetic elongated portion 2201 being connected to the prosthetic spherical portion 45. According to the embodiment shown in fig. 17a the elongated portion 2201 does not have any recesses or adaptations facilitating the movement of the prosthetic portions in relation to a prosthetic replacement for the acetabulum comprising extending portions.

Fig. 17b shows a prosthetic spherical portion 45 and a prosthetic elongated portion 2201 in a schematic view in an embodiment where the elongated portion comprises a recess 2203a adapted to further enable movement of the prosthetic replacement for the acetabulum 65 in relation to the prosthetic spherical 45 and elongated portions 2201.

Fig. 17c shows a prosthetic spherical portion 45 and a prosthetic elongated portion 2201 in a schematic view in an embodiment in which the elongated portion 2201 comprises two recesses 2203a,b adapted to further enable movement of the prosthetic replacement for the acetabulum 65 in relation to the prosthetic spherical 45 and elongated portions 2201.

Fig. 17d shows a prosthetic spherical portion 45 and a prosthetic elongated portion 2201 in a schematic view in an embodiment in which the elongated portion 2201 is curved such that the area in which the elongated portion 2201 connects to the spherical portion 45 is eccentrically placed in relation to the collum and caput femur center axis CX.

Fig. 18 shows the prosthetic parts replacing the articulating surfaces of the hip joint. The prosthetic replacement for the acetabulum surface 65 comprises extending portions 1823a,b which are adapted to enter a recess 2203 of the prosthetic elongated portion 2201. The recess enables further movement of the prosthetic replacement for the acetabulum 65 in relation to the prosthetic spherical 45 and elongated portions 2201 in the direction of recess, such as disclosed in the right part of fig. 5, where the prosthetic part is placed in a state in which the prosthetic replacement for the acetabulum 65 is moved maximally in the direction of the recess 2203.

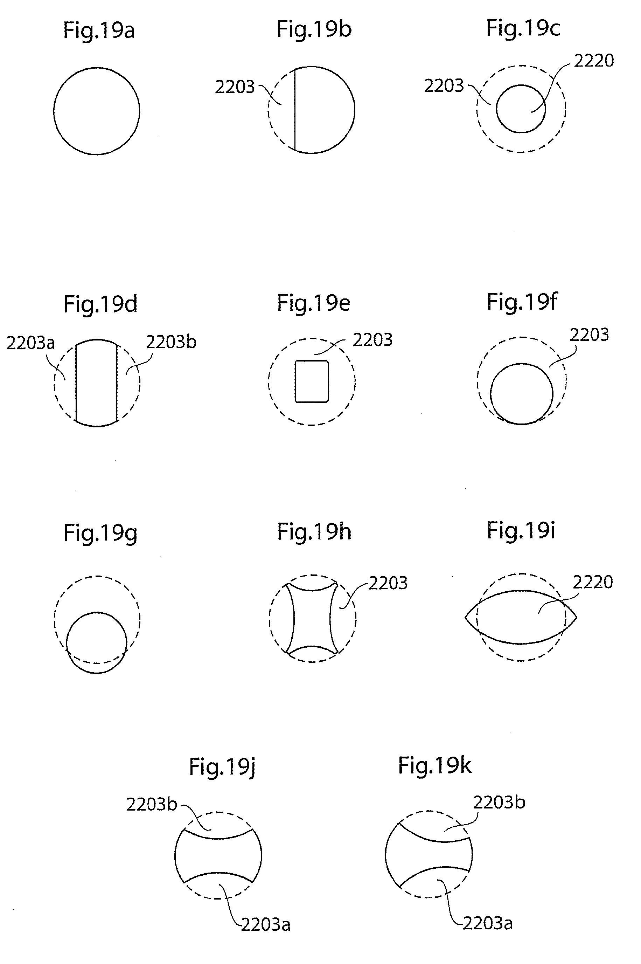

Fig. 19a shows a cross-section of the prosthetic elongated portion without an adaptation to increase to motion range. This embodiment is disclosed in a schematic side view in fig. 17a.

Fig. 19b shows a cross-section of the prosthetic elongated portion having a recess 2203 or adaptation such that said prosthetic replacement for the acetabulum 65 is adapted for further movement in relation to a prosthetic spherical 45 and elongated portion 2201 in the direction of the recess 2203 or adaptation. This embodiment is disclosed in a schematic side view in fig. 17b.

Fig. 19c shows a cross-section of the prosthetic elongated portion where the restricting part of the elongated prosthetic portion is a narrow portion 2220, with a smaller cross-sectional area than other portions of the elongated portion. In embodiments where the extending portions of a prosthetic replacement for the acetabulum extends circularly 360° around the collum and caput center axis, the circular narrow portion 2220 disclosed in fig. 19c enables further movement of the prosthetic replacement for the acetabulum 65 in all radial directions. This embodiment is disclosed in a schematic side view in fig. 17c.

Fig. 19d shows a cross-section of the prosthetic elongated portion comprising two recesses 2203a and 2203b or adaptations, into which a prosthetic replacement for the acetabulum can enter for further increasing the motion range of the prosthetic hip joint in the directions of the recesses or adaptations. This embodiment is disclosed in a schematic side view in fig. 17c.

Fig. 19e shows a cross-section of the prosthetic elongated portion wherein the restricting portion of the elongated portion has a narrow square cross-section such that for increasing the motion range of the prosthetic hip joint more in the directions of the sides of the square cross-section of the restricting portion of the elongated portion. This embodiment is disclosed in a schematic side view in fig. 17c.

Fig. 19f shows a cross-section of the prosthetic elongated portion wherein the restricting portion of the elongated portion is more narrow that other portions of the elongated portions and eccentrically placed in relation to the caput and collum center axis. The eccentric placement of the restricting portion of the elongated portion increases the motion range of the prosthetic hip joint in the direction of the recess created by the narrow portion being placed eccentrically. This embodiment is disclosed in a schematic side view in fig. 17b.

Fig. 19g shows a cross-section of the prosthetic elongated portion similar to the embodiment shown in fig. 6f. However in the embodiment shown in fig. 19g the narrow portion of the elongated portion is placed further eccentrically, further increasing the motion range of the prosthetic hip joint in the direction of the recess created by the narrow portion being placed eccentrically.

Fig. 19h shows a cross-section of the prosthetic elongated portion in which a potion of the elongated portion comprises a plurality of recesses 2203 which are adapted to a particular prosthetic replacement for the acetabulum. According to the embodiment shown in fig. 19h the restricting portion is adapted for a prosthetic replacement for the acetabulum comprising extending portions with a circular surface entering the recess of the elongated prosthetic portion.

Fig. 19i shows a cross-section of the prosthetic elongated portion, wherein said cross section is a more narrow elliptically shaped cross section 2220 which enables further movement of the prosthetic replacement for the acetabulum 65 in the narrow direction of the elliptically shaped elongated portion.

Fig. 19j shows a cross-section of the prosthetic elongated portion in which a potion of the elongated portion comprises two recesses 2203a,b which are adapted to a particular prosthetic replacement for the acetabulum. According to the embodiment shown in fig. 19h the restricting portion is adapted for a prosthetic replacement for the acetabulum comprising extending portions with a circular surface entering the recess of the elongated prosthetic portion.

Fig. 19k shows a cross-section of the prosthetic elongated portion in an embodiment similar to the embodiment sown in fig. 19j, however, in the embodiment of fig. 19k the recesses 2203a,b are somewhat eccentrically placed in relation to the caput and collum center axis. The eccentrically placed recesses 2203a,b thus altering the directions in which the motion range are further increased, such that the recesses could be adapted to more critical motions that a patient is interesting in doing.

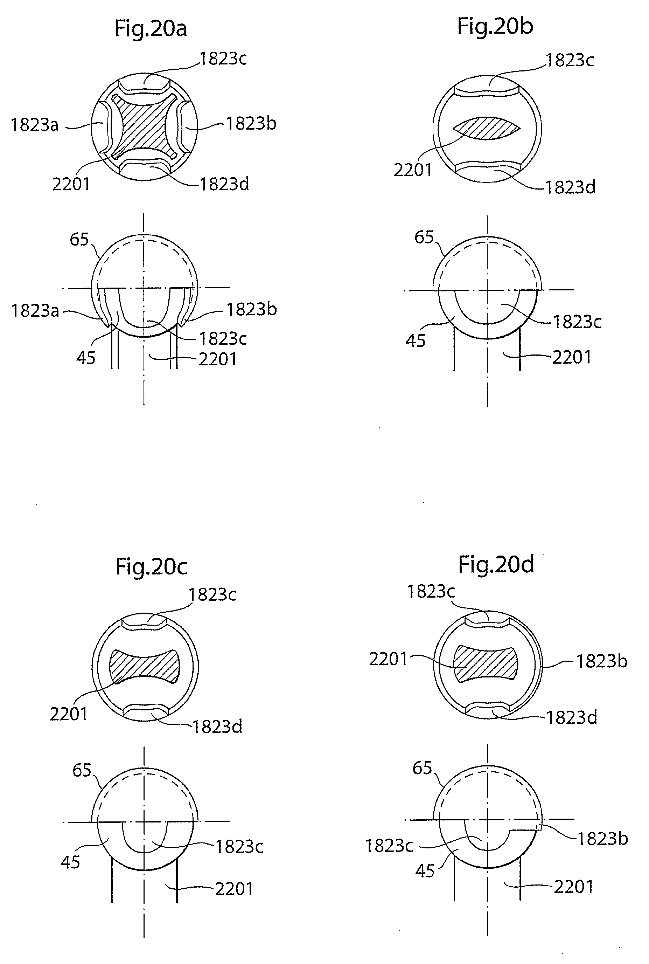

Fig. 20a shows the prosthetic elongated portion 2201, also shown in fig. 19h, when placed in the prosthetic replacement for the acetabulum 65 in a cross-sectional view, and in a side view. In the embodiment of fig. 20a, the prosthetic replacement for the acetabulum surface comprises four extending portions 1823a,b,c,d clasping the prosthetic spherical portion 45 when implanted in the functional position in the hip joint. Each of the extending portions 1823a,b,c,d of the prosthetic replacement for the acetabulum has a rounded shape adapted to match corresponding rounded recesses of the elongated portion 2201. By the extending portions 1823a,b,c,d of the prosthetic acetabulum 65 entering the rounded recesses of the elongated portion 2201 the motion range of the prosthetic replacement for the acetabulum 65 is further increased.

Fig. 20b shows the prosthetic elongated portion 2201, also shown in fig. 6i, when placed in the prosthetic replacement for the acetabulum 65 in a cross-sectional view, and in a side view. In the embodiment of fig. 20b, the prosthetic replacement for the acetabulum surface comprises two rounded extending portions 1823c,d clasping the prosthetic spherical portion 45 when implanted in the functional position in the hip joint. The cross section of the prosthetic elongated portion is a more narrow, elliptically shaped cross section which enables further movement of the prosthetic replacement for the acetabulum 65 in the narrow direction of the elliptically shaped elongated portion, where according to this embodiment, the extending portions of the prosthetic acetabulum is placed.

Fig. 20c shows the prosthetic elongated portion 2201, also shown in fig. 6j, when placed in the prosthetic replacement for the acetabulum 65 in a cross-sectional view, and in a side view. In the embodiment of fig. 20c, the prosthetic replacement for the acetabulum surface comprises two rounded extending portions 1823c,d clasping the prosthetic spherical portion 45 when implanted in the functional position in the hip joint. The two extending portions 1823c,d of the prosthetic replacement for the acetabulum has a rounded shape adapted to match corresponding rounded recesses of the elongated portion 2201. The shape of the prosthetic replacement for the acetabulum 65 is so adapted that the prosthetic replacement for the acetabulum can move along a relatively large motion range whilst the extending portions still clasping the prosthetic spherical portion 45.

Fig. 20d shows the prosthetic elongated portion 2201, also shown in fig. 19k, when placed in the prosthetic replacement for the acetabulum 65 in a cross-sectional view, and in a side view. In the embodiment of fig. 20d, the prosthetic replacement for the acetabulum surface comprises two rounded extending portions 1823c,d , and one circumferentially elongated extending portion 1823b clasping the prosthetic spherical portion 45 when implanted in the functional position in the hip joint. The two extending portions 1823c,d of the prosthetic replacement for the acetabulum has a rounded shape adapted to match corresponding rounded recesses of the elongated portion 2201. The third extending portion 1823b is not extending as far as the two 1823c and 1823d, thus not limiting the motion range as much. The shape of the prosthetic replacement for the acetabulum 65 is so adapted that the prosthetic it can move along a relatively large motion range whilst the extending portions still clasping the prosthetic spherical portion 45.

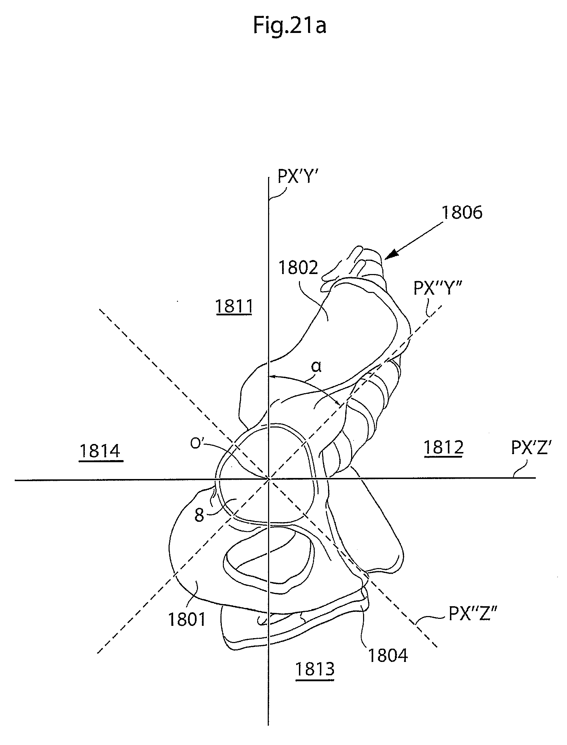

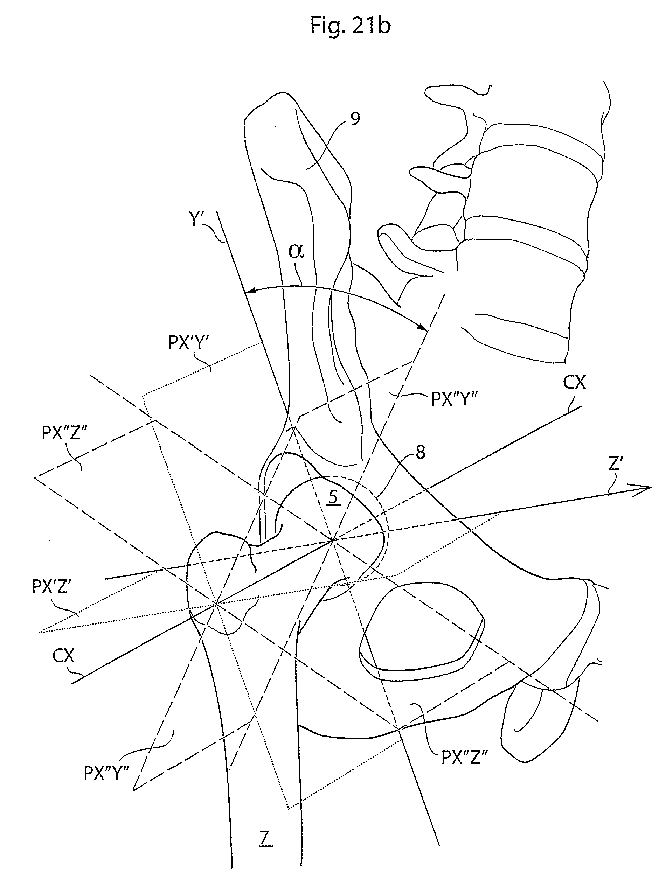

Fig. 21 a shows pelvis in the same view as fig. 2b. Here the vertical and horizontal acetabulum planes PX'Y' and PX'Z' (further disclosed with reference to fig. 2d) are shown in a strict plane view. Two further planes PX"Y" is introduced in fig. 3, which planes are rotated an angle α of 45° clockwise. The planes PX"Y" and PX"Z", in accordance with the planes PX'Y' and PX'Z' divides the acetabulum bowl into four different quadrants, being a proximal quadrant 1811, a frontal quadrant 1812, a distal quadrant 1813 and a dorsal quadrant 1814.

Fig. 21b shows a perspective view of the hip joint in section, displaying the horizontal and vertical planes PX'Y' and PX'Z' originating from the caput 5 and collum 6 center axis CX and dividing the caput 5 and collum 6 femur into quadrants. Fig. 21b further shows the planes PX"Y" and PX"Z" dividing the acetabulum bowl into four different quadrants, being a proximal quadrant 1811, a frontal quadrant 1812, a distal quadrant 1813 and a dorsal quadrant 1814, which is further disclosed with reference to fig. 3a.

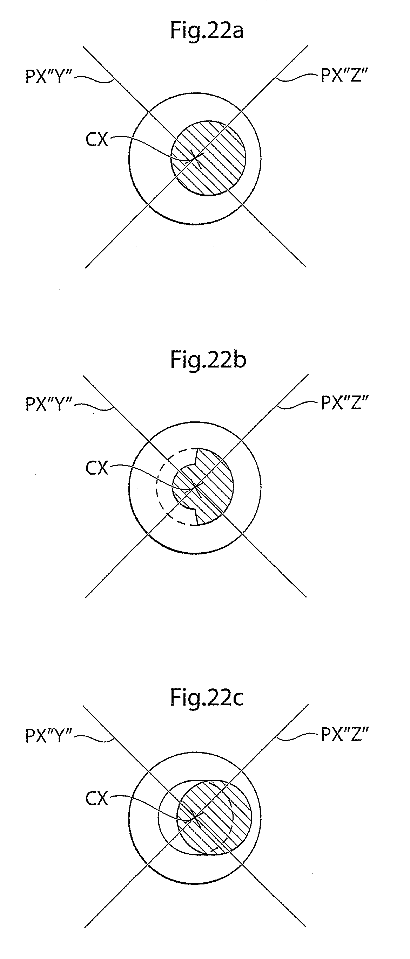

Fig. 22a shows a cross-section of an embodiment of the prosthetic elongated portion 2201, wherein said elongated portion is eccentrically placed in relation to the collum and caput center axis CX. The eccentric placement of the elongated portion 2201 places a large portion of the elongated portion 2201 a in the dorsal quadrant, smaller portions of the elongated portions 2201 b,d in the proximal and distal quadrants, respectively, and a.smaller portion 2201c is placed in the frontal quadrant. The placing of a larger portion of the restricting portion of the elongated portion 2201 in the dorsal quadrant 1814 limits the motion range in the extension mostly. The movement range of the extension movements is less critical than for example flexion for every day activities, thus placing the major part of the elongated portion 2201 a in the dorsal quadrant restricts the motion range needed in a less critical way. The eccentrically placed elongated portion is further disclosed with reference to fig. 19f and 19g.

Fig. 22b shows a cross-section of an embodiment of the prosthetic elongated portion 2201, wherein said elongated portion comprises a recess 2203, mainly placed in the frontal 1812, proximal 1811 and distal 1813 quadrant, thus placing the major portion of the restricting portion of the elongated member in the dorsal quadrant 1814. The placing of a larger portion of the restricting portion of the elongated portion 2201 in the dorsal quadrant 1814 limits the motion range in the extension mostly. The movement range of the extension movements is less critical than for example flexion for every day activities, thus placing the major part of the elongated portion 2201 a in the dorsal quadrant restricts the motion range needed in a less critical way. The elongated portion comprising a recess is further disclosed with reference to fig. 17b and 19b.

Fig. 22c shows a cross-section of an embodiment of the prosthetic elongated portion 2201, wherein said elongated portion 2201 is curved such that the connecting area between the prosthetic spherical portion 45 and the prosthetic elongated portion 2201 is eccentrically placed in relation to the caput and collum center axis CX., thus placing the major portion of the restricting portion of the elongated member 2201 in the dorsal quadrant 1814. The placing of a larger portion of the restricting portion of the elongated portion 2201 in the dorsal quadrant 1814 mainly limits the motion range in extension of the leg. The movement range of the extension movements is less critical than for example flexion for every day activities, thus placing the major part of the elongated portion 2201 a in the dorsal quadrant restricts the motion range needed in a less critical way. The elongated portion comprising the curved elongated portion is further disclosed with reference to fig. 17d.

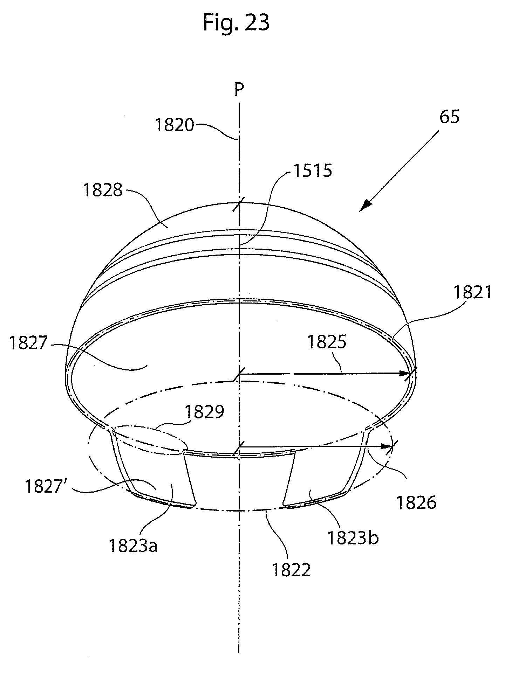

Fig. 23 shows a medical device for implantation in a hip joint of a patient. The medical device is adapted to be fixated to the femoral bone of the patient, for example by means of an adhesive, such as bone cement, or mechanical fixating members, such as orthopedic screws. The medical device comprises an inner 1827 and an outer 1828 surface. A contacting portion of the inner surface 1827 is spherical and faces the center of the hip joint, when the medical device is implanted. The inside of the medical device is adapted to receive a prosthetic replacement for a caput femur having a spherical portion, and the spherical contacting portion of the inner surface 1827 is adapted to be in contact with a spherical portion of the outer surface of the prosthetic replacement of the caput femur. The medical device, according to the embodiment shown in fig. 17 comprises two extending portions 1823a,b, extending the contacting portion of the inner surface 1827' such that the extending portions 1823a,b clasps the spherical portion of a prosthetic replacement of a caput femur, for restraining the spherical portion in the medical device. The medical device is adapted to receive the prosthetic spherical portion, connected to the prosthetic elongated portion. The inner surface 1827 comprises an equator line 1821, being the largest circular circumference of the inner surface. The two extending portions passes beyond the equator line 1821, such that an end portion 1829 of the contacting portion, here being of the extending portion 1823b of the inner surface 1827, forms a circular extension line 1822 placed proximal to the equator line 1821, when the medical device is implanted, and having a smaller circumference than the equator line 1821; thus a distance 1826 between a center axis P of the medical device and the extension line 1822 is shorter than a distance 1825 between the center axis P and the equator line 1821.

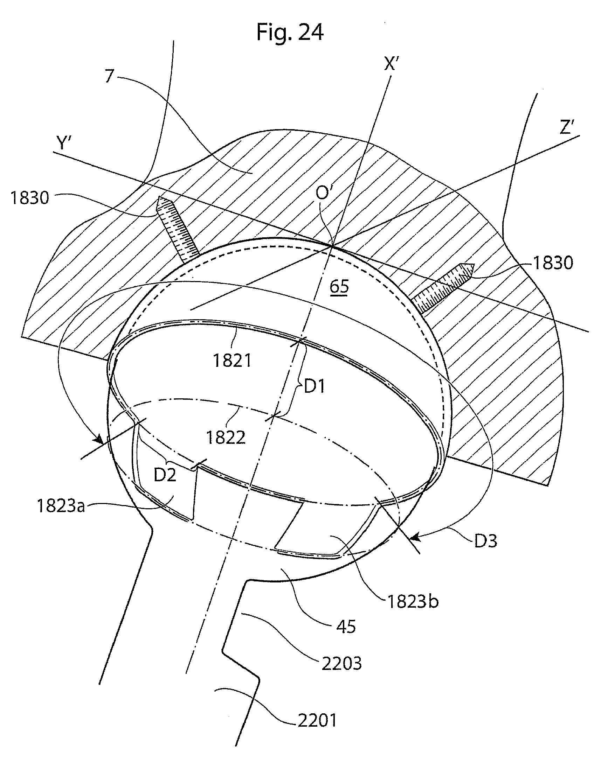

Fig. 24 shows the medical device described with reference to fig. 23 when implanted. According to this embodiment the medical device is adapted to be fixated using orthopedic screws 1830, mechanically fixating the medical device to the femoral bone 5, by the medical device comprising holes through which the screws 1830 are placed. In fig. 5 the contacting portion of the inner surface 1827 has been placed in contact with the prosthetic spherical portion being connected to a the prosthetic elongated portion 2201, the prosthetic spherical 45 and elongated portions 2201 replacing the proximal portion of the femoral bone. The two extending portions 1823a and 1823b extending the contacting portion of the inner surface and clasping the spherical portion 45, for restraining the spherical portion in the medical device. The inner surface comprising the equator line 1821, and the extending portions 1823a,b passing beyond the equator line 1821 and comprising the more proximal extension line 1822 having a smaller circumference than the equator line 1821. The more proximal extension line 1822 being placed at a distance D1 from the equator line 1821. According to this embodiment the extension line 1822 is parallel to the equator line 1821, however this is not necessarily so in other embodiments. The extension portion 1823a according to the embodiment shown in fig. 11 extends circumferentially along the equator line, a distance D2. Along another portion of the equator line, a distance D3, there are no extending portion, which enables the elongated portion 2201 to enter the space between the first and second extending portions 1823a,b which creates a larger movement range of the hip joint, for further increase of the movement range, the recess 2203 in the elongated portion 2201 is adapted for some section of the extending portion to enter the recess 2203.

The extending portion, according to any of the embodiments, adapted to clasp the prosthetic spherical portion, for restraining it the prosthetic acetabulum 65, could further be adapted to release the prosthetic spherical portion 45 when a large enough strain is placed on the joint. This feature enables the prosthetic spherical portion to be fixedly attached in the prosthetic acetabulum 65 in normal use, and be released from the prosthetic acetabulum, e.g. in case of an accident, thus reducing the risk of damaging the bodily structures, such as the femoral bone, or the fixations between bodily structures and prosthetic parts.

According to one embodiment, the extending elements, are placed such that the extending elements restricts the motion range minimally, or in ways which are not limiting the motion range used in everyday life. This is enabled through the placing of the extending portions, or the interaction between the extending portion and adaptations of the prosthetic elongated portion. The hip joint is a synovial ball and socketjoint which permits a large motion range for allowing a plurality of different movements of the lower limb. From a neutral position, the following movements of the hipjoint are normally possible: Lateral or external rotation, 30° with the hip extended, 50° with the hip flexed, medial or internal rotation 40°, extension or retroversion 20°, flexion or anteversion 140°, abduction 50° with hip extended, 80° with hip flexed, adduction 30° with hip extended, 20° with hip flexed. In the movement ranges of abduction and adduction the depth of the acetabulum bowl and thus the extending portions does not restrict the motion range in a critical way since the motion range of the normal hip is restricted in these movements, in normally agile persons, by the muscles, tenors and ligaments surrounding the hipjoint.

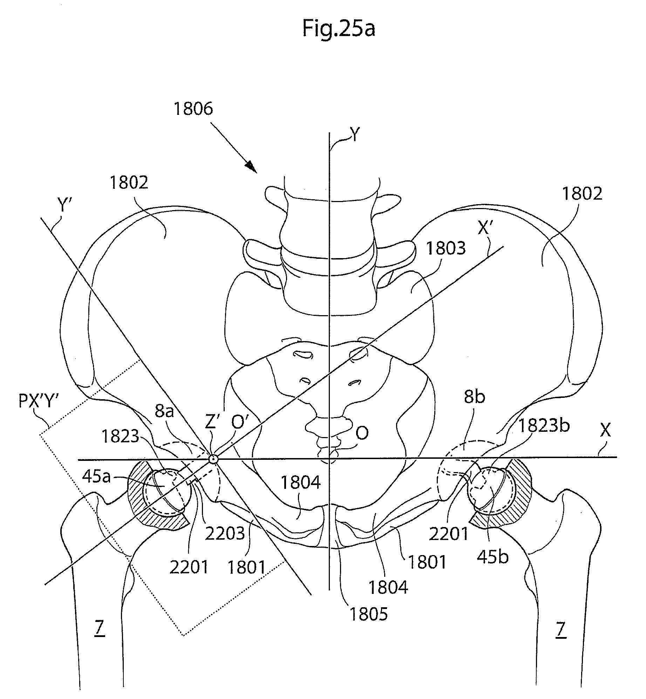

Fig. 25a shows a frontal view of pubis and the proximal portions of the femoral bones 7 when two embodiments prosthetic replacement for the acetabulum 65 has been implanted in the hipjoint. The prosthetic replacements for the acetabulum shown comprises one extending portion 1823, here placed dorsal to the vertical acetabulum plane PX'Y' in the base position, thus only partially limiting abduction in far excess of 50°. According to the embodiment shown, the extending portion 1823 extends circumferentially along the equator line 1821 about 1/10 of the length of the equator line 1821, however in other embodiments the extending portion 1823 extends along as much as half of the length of the equator line 1821, and in other embodiments the extending portion 1823 extends as little as about 1/30 of the length of the equator line 1821. The prosthetic replacement for the acetabulum placed in the left femoral bone comprises two extending portions 1823a,b, both being placed dorsal the corresponding vertical acetabulum plane PX'Y' of the left acetabulum (not shown) in the base position, thus limiting the motion range of the hipjoint in a non restrictive way, in relation to everyday activities. In both the right and left embodiment the extending portions 1823 extends discontinuously along the equator line 1821 thus enabling the elongated portion 2201 to partially be placed between the equator line and the extension line, and in the left embodiment, be placed between the extending portions 1823a,b thus entering the cavity between the extending portions 1823a,b. The recess 2203 of the prosthetic elongated portion 2201 implanted in the right hip joint is radially placed, in relation to the caput and collum center axis, such that the a section of the prosthetic elongated portion 2201, can enter the recess for further increasing the movement range of the prosthetic acetabulum surface 65 in relation to the elongated 2201 and spherical 45a portion. The curving of the prosthetic elongated portion 2201 implanted in the left hipjoint is radially placed, in relation to the caput and collum center axis, for further increasing the movement range of the prosthetic acetabulum surface 65 in relation to the elongated 2201 and spherical 45b portion.

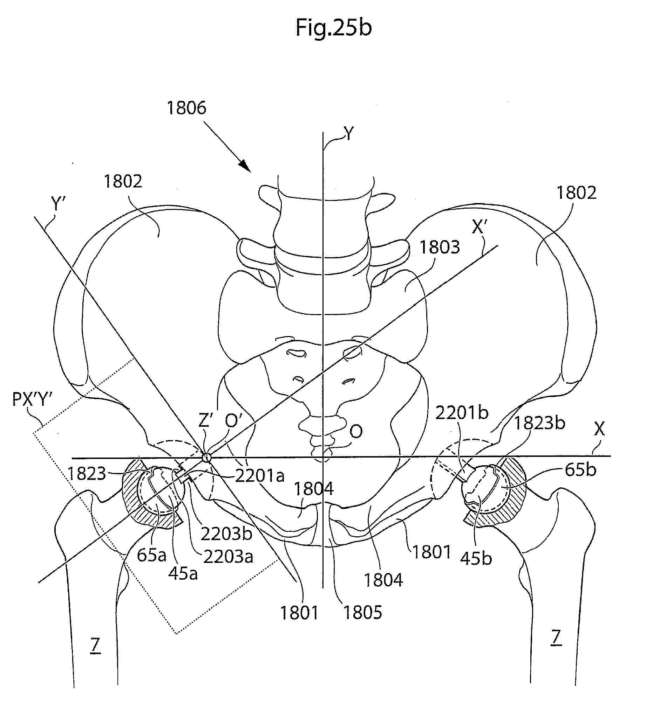

Fig. 25b shows a frontal view of pubis and the proximal portions of the femoral bones 7, when two further embodiments of the prosthetic replacements have been implanted. The embodiment shown placed on the right side is an embodiment in which the prosthetic elongated portion 2201 a comprises a first 2203a and second 2203b recess placed at the restricting portion of the elongated portion 2201 a. The prosthetic elongated portion is connected to a prosthetic spherical portion 45a which is restrained in a prosthetic replacement for the acetabulum 65a fixated to the femoral bone. The prosthetic replacement for the acetabulum 65a comprises extending portions 1823 clasping the prosthetic spherical portion 45a and thus restraining the spherical portion in the prosthetic replacement for the acetabulum 65a. The extending portions 1823 is placed in the proximal quadrant in the base position, thus limiting the motion range of the hip joint in a non restrictive way, in relation to everyday activities. According to the embodiment shown, the extending portion 1823 extends circumferentially along the equator line 1821 about 1/10 of the length of the equator line 1821, however in other embodiments the extending portion 1823 extends along as much as half of the length of the equator line 1821, and in other embodiments the extending portion 1823 extends as little as about 1/30 of the length of the equator line 1821. The prosthetic elongated portion 2201 b shown placed in the left hip joint comprises a narrow portion connected to the prosthetic spherical portion 45b. The narrow portion enables a relatively large motion range in relation to the elongated portion even though the prosthetic replacement for the acetabulum comprises extending portions 1823a,b extending beyond the equator line of the prosthetic spherical portion 45b, thus clasping the spherical portion and restraining it in a fixated position.

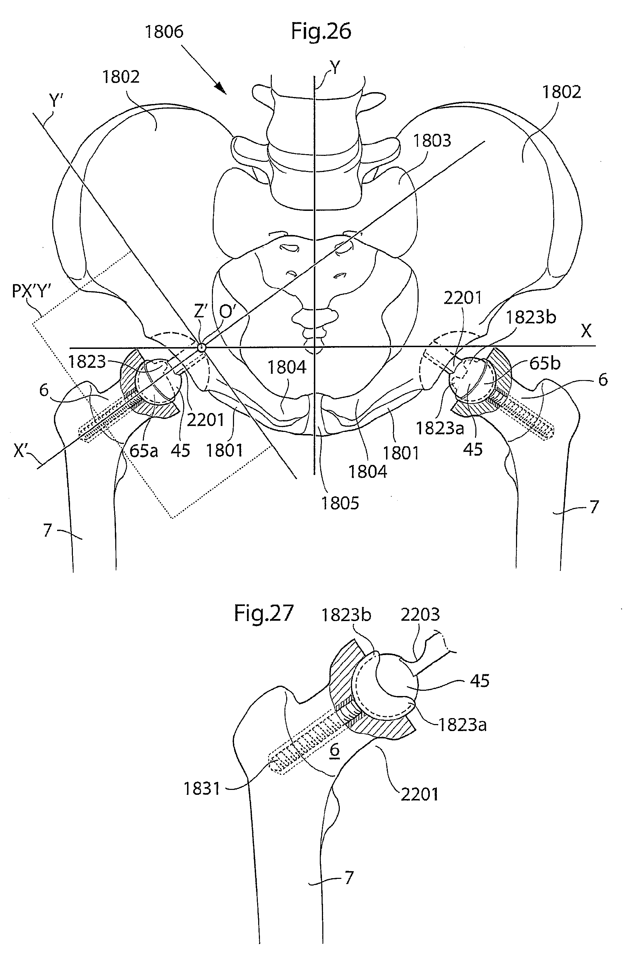

Fig. 26 shows the pelvis and the proximal portions of the femoral bones 7 including the embodiment of fig. 25a, with the difference that the natural caput femur and a portion of the natural collum femur has been replaced by a prosthetic replacement for the acetabulum. The prosthetic elongated portion 2201 is here coordinated with the extending portions 1823 of the prosthetic replacement for the acetabulum 65a,b for further improving the motion range of the hip joint, or not limiting the natural motion range of the hip joint.

Fig. 27 shows the medical device according to an embodiment in which the medical device comprises two extending portions 1823a,b. The medical device is placed on a prosthetic elongated portion 2201, to which a prosthetic spherical portion 45 is attached. The prosthetic elongated member 2201 is here adapted to further improve the motion range of the hip joint, or not limiting the natural motion range of the hip joint, by the prosthetic elongated portion 2201 comprising a recess 2203 in which the extending portions 1823 can enter.

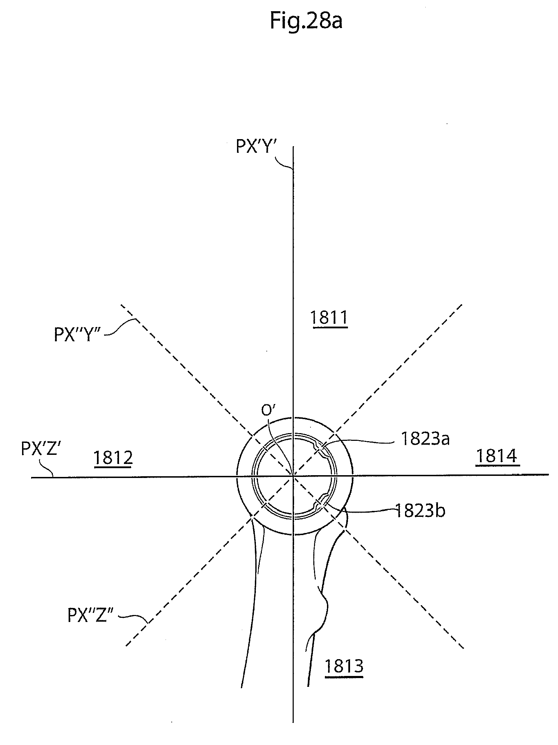

Fig. 28a shows the pelvis in a lateral view, the prosthetic replacement for the acetabulum 65 in the femoral bone comprises two extending portions 1823a,b, both extending circumferentially along the equator line (as disclosed in for example fig. 5) dorsal to the caudal-cranial axis Y when being in the base position and being adapted to clasp the caput femur or a prosthetic replacement therefor. The extending portions 1823a,b extending dorsal to the caudal-cranial axis Y when being in the base position and thus reducing the limiting effect that the extending portions 1823a,b, have on the motion range of the hip joint. According to the embodiment shown in fig. 15a the extending portion 1823a, placed proximally in the acetabulum, extends circumferentially a distance of about ¼ of the length of the equator line, and the extending portion 1823b, placed distally in the acetabulum when being in the base position, extends circumferentially a distance of about 1/10 of the length of the equator line, however it is equally conceivable that this relationship is the other way around, or that any of the extending portions circumferentially extends a distance of as much as half of the length of the equator line, thus extending the entire distance of the equator line being dorsal to the vertical acetabulum plane PX'Y', or that any of the extending portions 1823a,b extends a distance being as little as 1/30 of the distance of the equator line. According to the embodiment shown in fig. 15a, the first extending portion 1823a extends in distal-lateral direction from the acetabulum, and the second extending portion 1823b extends medially towards foramen obturatum when being in the base position.

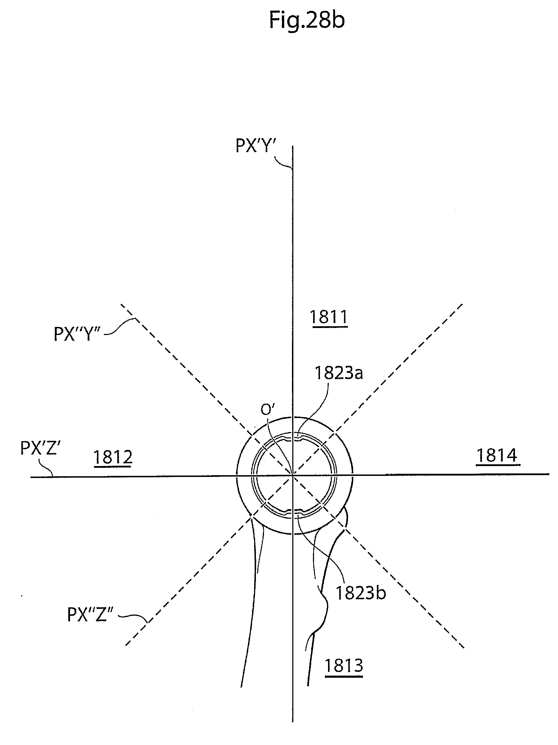

Fig. 28b shows the pelvis in a lateral view, the prosthetic replacement for the acetabulum 65 in the femoral bone comprises two extending portions 1823a,b, the two extending portions 1823a,b extends in the proximal quadrant 1811 and the distal quadrant 1813 when being in the base position, respectively.

There are multiple ways in which the extending portions 1823 can be adapted to reduce the effects that the extensions have on the motion range of the hip joint.

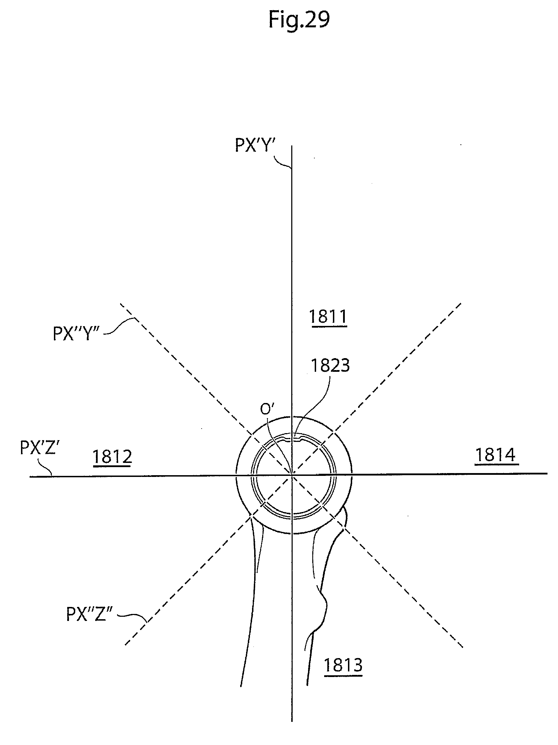

Fig. 29 shows the pelvis in a lateral view, the prosthetic replacement for the acetabulum 65 in the femoral bone shown comprises one extending portion 1823 extending and being adapted to clasp the caput femur, or a prosthetic replacement therefor. The extending portion 1823 extends circumferentially along the equator line within the proximal quadrant 1811 when being in the base position, which is further disclosed with reference to fig. 3. According to the embodiment shown in fig. 10, the extending portion 1823 extends in distal-lateral direction from the acetabulum when being in the base position.

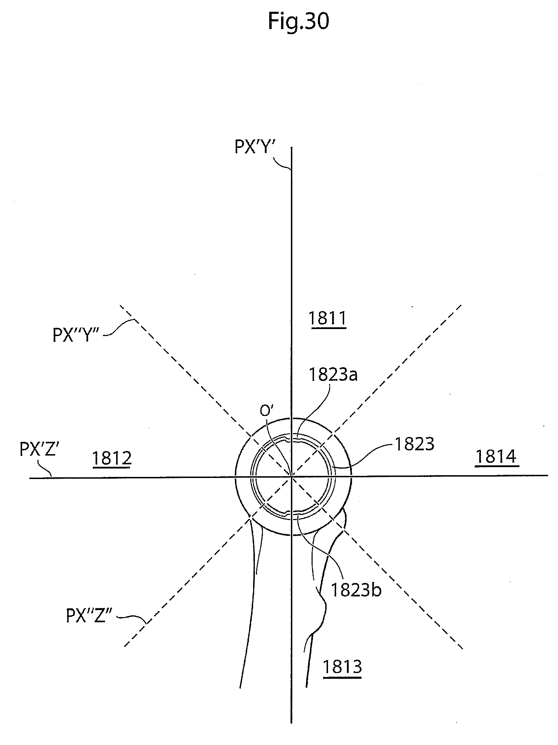

Fig. 30 shows the pelvis in a lateral view, the prosthetic replacement for the acetabulum 65 in the femoral bone shown comprises a continuously extending portion 1823 with two extending portions 1823a and 1823b extending further in relation to the average extension of the extending portion. The entire extending portion is placed in the proximal, distal and dorsal quadrants and the extending portions 1823a,b extending further than the average extension of the extending portion 1823 extends in the proximal and distal quadrant when being in the base position.

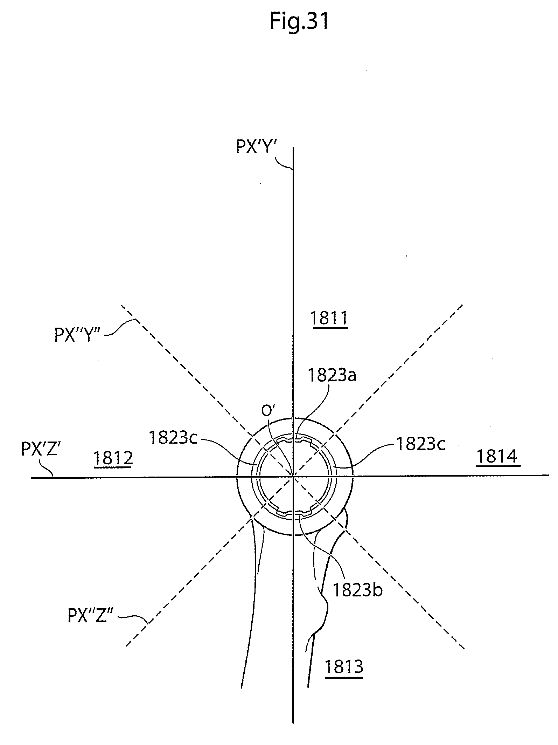

Fig. 31 shows the pelvis in a lateral view, the prosthetic replacement for the acetabulum 65 in the femoral bone shown comprises four extending portions 1823a,b,c,d, wherein the first 1823a and second 1823b extending portions extends in the proximal and distal quadrant, respectively, thus the first extending portion 1823a extending in distal-lateral direction from the acetabulum, and the second extending portion 1823b extending medially towards foramen obturatum when being in the base position. The third extending portion 1823c when being in the base position extending in the frontal quadrant 1812, out from the acetabulum in dorsal direction, extends less than the first and second extending portion, since extending portions 1823c in the frontal quadrant is more limiting to the normal motion range of the hip joint. The fourth extending portion 1823d extends in the dorsal quadrant in accordance with the third extending portion 1823c do not extend as far as the first and second extending portions when being in the base position.

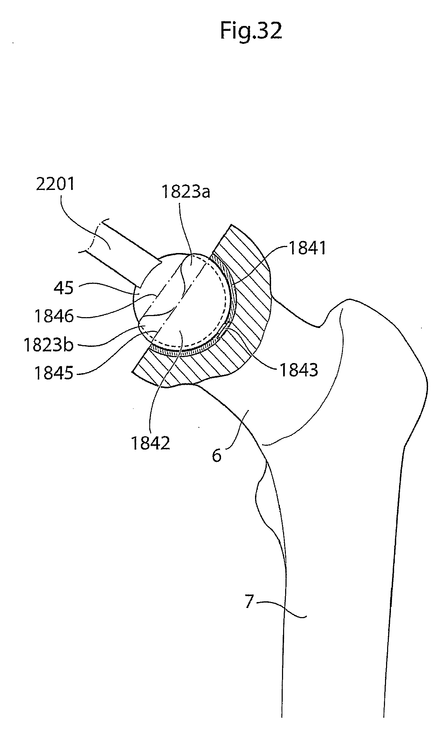

Fig. 32 shows an alternative embodiment of the prosthetic replacement for the acetabulum 65. In the alternative embodiment, the prosthetic replacement for the acetabulum 65 comprises a first part 1841 adapted to be fixated to the femoral bone of the patient. The first part comprises an inner contacting surface adapted to be in movable connection with an outer contacting surface of a second part 1842. The second part 1842 is rotatably fixated to the first part 1841 by a rotatable connecting member 1843. An outer contacting surface of a prosthetic spherical portion 45 is adapted to be placed in contact with the inner surface of the second part 1842 and be movable in multiple directions, thus replicating the natural ball and socket joint of the hip. The second part 1842 comprises two extending portions 1823a,b extending beyond the equator line 1845 of the second part 1842. The extending portions 1823a,b extends longitudinally discontinuously along the equator line, thus creating an area between the extending portions, in which area a portion of the prosthetic collum femur can be placed, thus being placed partially between the equator line 1845 and the extension line 1846. The construction shown in fig. 26 enables the second part 1842 to rotate if the prosthetic elongated portion 2201 engages the extending portions 1823a,b, which are sloped for this purpose. This way the second part 1842 are always placed such that the prosthetic elongated portion 2201 can be placed partially between equator line 1845 and the extension line 1846, which creates an optimal range of movement whilst the second part clasps the prosthetic spherical portion 45, and thus restricting the spherical portion 45 in the second part 1842 of the prosthetic replacement for the acetabulum 65.

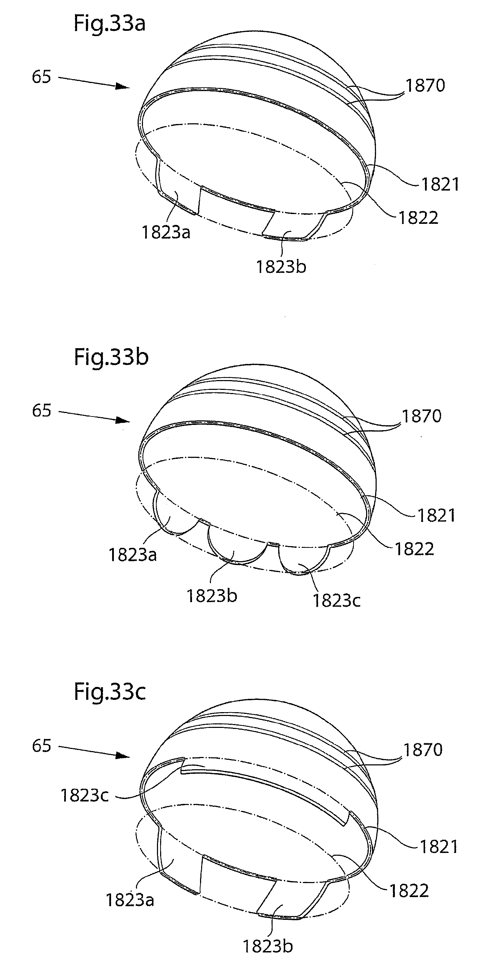

Fig. 33a shows the prosthetic replacement for the acetabulum 65 according to one embodiment. In this embodiment the prosthetic replacement for the acetabulum 65 comprises two extending portions 1823a, b. The prosthetic replacement for the acetabulum 65 is according to this embodiment adapted to be fixated to the femoral bone by means of an adhesive which is adapted to be placed in connection with the adhesive recesses 1870 of the outer surface of the prosthetic replacement for the acetabulum 65.

Fig. 33b shows a prosthetic replacement for the acetabulum 65 similar to the prosthetic replacement for the acetabulum 65 disclosed with reference to fig. 33a, but with the difference that it comprises three equally extending portions 1823a,b,c.

Fig. 33c shows a prosthetic replacement for the acetabulum 65, similar to the prosthetic replacement for the acetabulum 65 disclosed with reference to fig. 33a, but with the difference that it comprises two equally extending portions 1823a,b and one less extending portion 1823c.

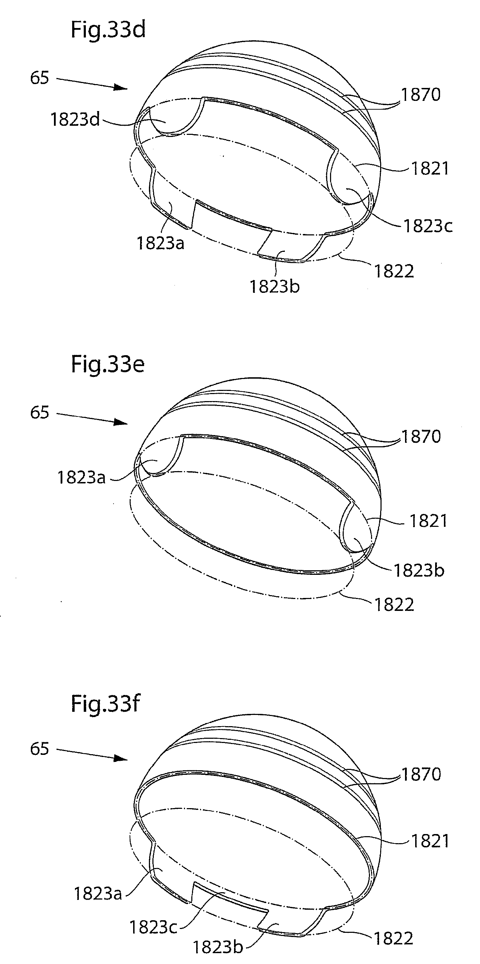

Fig. 33d shows a prosthetic replacement for the acetabulum 65 similar to the prosthetic replacement for the acetabulum 65 disclosed with reference to fig. 33a, but with the difference that it comprises four equally extending portions 1823a,b,c,d.

Fig. 33e shows a prosthetic replacement for the acetabulum 65 similar to the prosthetic replacement for the acetabulum 65 disclosed with reference to fig. 33a, but with the difference that the two extending portions are placed further from each other, and thus being adapted to be placed in the proximal and distal quadrant, when implanted and being in the base position.

Fig. 33f shows a prosthetic replacement for the acetabulum 65 similar to the prosthetic replacement for the acetabulum 65 disclosed with reference to fig. 33a, but further comprising a less extending portion 1823c placed between the first and second extending portions 1823a,b.

The extending portions of the prosthetic replacement for the acetabulum 65 which have been described could be made from an elastic material, enabling the extending portions to pass onto the a prosthetic spherical portion, according to any of the embodiments herein.

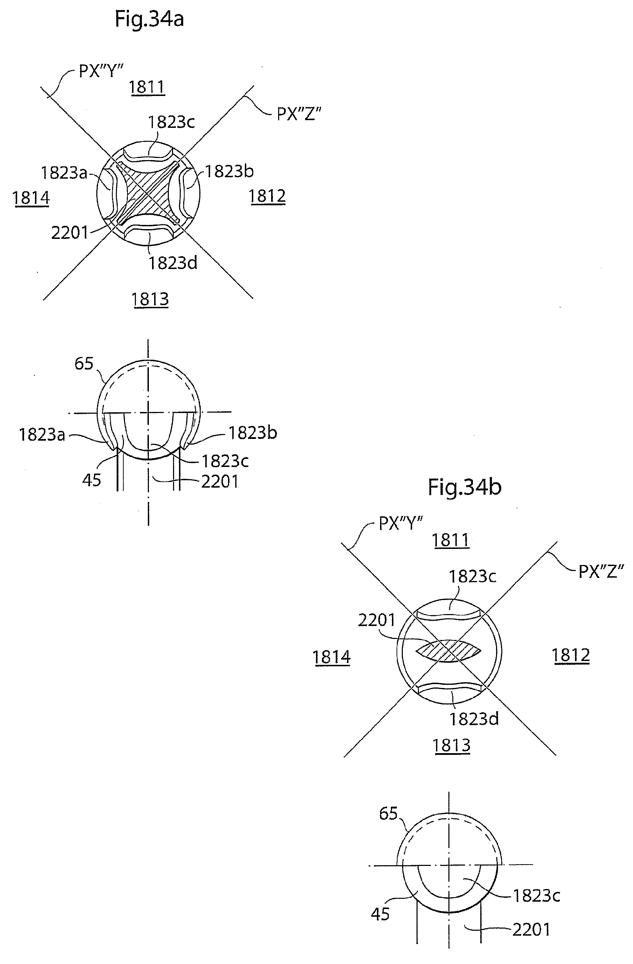

Fig. 34a shows the prosthetic elongated portion 2201, also shown in fig. 7a, when placed in the prosthetic replacement for the acetabulum 65 in a cross-sectional view, and in a side view. In the embodiment of fig. 34a, the prosthetic replacement for the acetabulum surface comprises four extending portions 1823a,b,c,d clasping the prosthetic spherical portion 45 when implanted in the functional position in the hip joint. Each of the extending portions 1823a,b,c,d of the prosthetic replacement for the acetabulum has a rounded shape adapted to match corresponding rounded recesses of the elongated portion 2201. By the extending portions 2823a,b,c,d of the prosthetic acetabulum 65 entering the rounded recesses of the elongated portion 2201 the motion range of the prosthetic acetabulum 65 is further increased. According to the embodiment shown in fig. 34a, the elongated portion 2201 is placed centered in relation to the collum and caput center axis, such that the an equal portion of the elongated portion is placed in the proximal 1811, distal 1813, frontal 1812 and dorsal 1814 quadrants, respectively.

Fig. 34b shows the prosthetic elongated portion 2201, also shown in fig. 20b, when placed in the prosthetic replacement for the acetabulum 65 in a cross-sectional view, and in a side view. In the embodiment of fig. 34b, the prosthetic replacement for the acetabulum surface comprises two rounded extending portions 1823c,d clasping the prosthetic spherical portion 45 when implanted in the functional position in the hip joint. The cross section of the prosthetic elongated portion is a more narrow, elliptically shaped cross section which enables further movement of the prosthetic replacement for the acetabulum 65 in the narrow direction of the elliptically shaped elongated portion, where according to this embodiment, the extending portions of the prosthetic acetabulum is placed. The prosthetic replacement for the acetabulum 65 and the prosthetic elongated portion 2201 are oriented in relation to the acetabulum, such that the extending portions 1823c,d are placed in the proximal 1811 and distal 1813 quadrant when being in the base position, and the major parts of the elongated portion 2201 are placed in the frontal 1812 and dorsal 1814 quadrants, while only minor parts are placed in the proximal 1811 and distal 1813 quadrants. This configuration enables the prosthetic replacement for the acetabulum 65 to have extending portions 1823c,d" and the elongated portion to have a relatively large cross-section without restricting the motion range of the hip joint in excess of the restriction of a natural hip joint, in any direction.

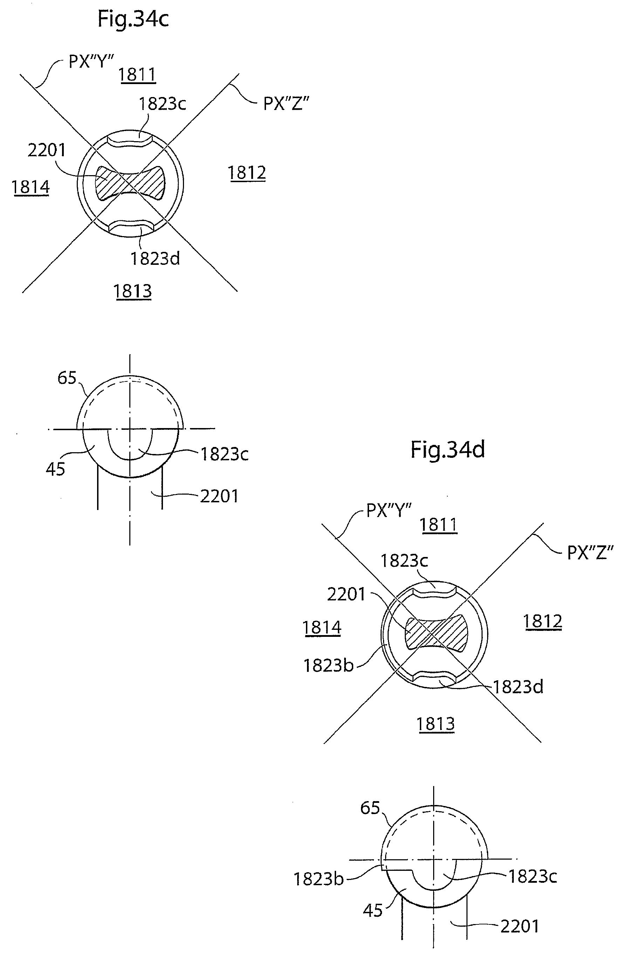

Fig. 34c shows the prosthetic elongated portion 2201, also shown in fig. 20c, when placed in the prosthetic replacement for the acetabulum 65 in a cross-sectional view, and in a side view. In the embodiment of fig. 21c, the prosthetic replacement for the acetabulum surface comprises two rounded extending portions 1823c,d clasping the prosthetic spherical portion 45 when implanted in the functional position in the hip joint. The two extending portions 1823c,d of the prosthetic replacement for the acetabulum 65 has a rounded shape adapted to match corresponding rounded recesses of the elongated portion 2201. The shape of the prosthetic replacement for the acetabulum 65 is so adapted that the prosthetic replacement for the acetabulum 65 can move along a relatively large motion range whilst the extending portions still clasping the prosthetic spherical portion 45. This configuration enables the prosthetic replacement for the acetabulum 65 to have extending portions 1823c,d, and the elongated portion to have a relatively large cross-section without restricting the motion range of the hip joint in excess of the restriction of a natural hip joint, in any direction.

Fig. 34d shows the prosthetic elongated portion 2201, also shown in fig. 20d, when placed in the prosthetic replacement for the acetabulum 65 in a cross-sectional view, and in a side view. In the embodiment of fig. 34d, the prosthetic replacement for the acetabulum 65 comprises two rounded extending portions 1823c,d , and one circumferentially elongated extending portion 1823b clasping the prosthetic spherical portion 45 when implanted in the functional position in the hip joint. The two extending portions 1823c,d of the prosthetic replacement for the acetabulum 65 has a rounded shape adapted to match corresponding rounded recesses of the elongated portion 2201. The third extending portion 1823b is not extending as far as the two 1823c and 1823d, thus not limiting the motion range as much. The shape of the prosthetic replacement for the acetabulum 65 is so adapted that the prosthetic replacement for the acetabulum 65 can move along a relatively large motion range whilst the extending portions still clasping the prosthetic spherical portion 45. The prosthetic replacement for the acetabulum 65 and the prosthetic elongated portion 2201 are oriented in relation to the acetabulum, such that the extending portions 1823c,d are placed in the proximal 1811 and distal 1813 quadrant when being in the base position, where only minor parts of the elongated portion 2201 are placed. A large portion if the elongated portion 2201 is placed in the dorsal quadrant 1814, where no extending portion is present, while a smaller, but still relatively large portion of the elongated portion 2201 is placed in the frontal 1812 quadrants, where a less extending portion is placed. This configuration enables the prosthetic replacement for the acetabulum 65 to have extending portions 1823c,b,d in three directions, creating a very stable configuration, whilst still enabling the elongated portion to have a relatively large cross-section without restricting the motion range of the hip joint in far excess of the restriction of a natural hip joint.

Examples of embodiments of different adaptations of the elongated portion, alone, or adaptations in conjunction with corresponding adaptations of the prosthetic replacement for the acetabulum have been shown. The placement of the elongated member in relation to the prosthetic replacement for the acetabulum is critical to the obtained motion range for a prosthetic hip joint comprising a prosthetic replacement for the acetabulum with portions extending beyond the equator of the spherical portion clasped therein. According to some embodiments 1/10 of the cross section of the restricting portion of the elongated member is placed in the proximal and distal quadrants, whereas 9/10 are placed in the frontal and dorsal quadrants, which 9/10 could be equally distributed between the two quadrants, or a major part, such as 6/10 could be placed in the dorsal quadrant and 4/10 could be placed in the frontal quadrants, however it is equally conceivable that it is the other way around depending of the design of the prosthetic replacement for the acetabulum. In embodiments comprising deep recesses or adaptations, or in embodiments where the restricting portion of the elongated portion is placed eccentrically in relation to the collum and caput center axis, as little as 0/10 of the elongated portion could be placed in a particular quadrant and from that the entire range from 1/10 - 10/10 of the restricting portion of the elongated portion could be placed in any of the quadrants to obtain an advantageous motion range, all depending on the placement of the prosthetic replacement for the acetabulum in relation to the femoral bone, and in particular the extending portions of the prosthetic replacement for the acetabulum when being in the base position.

The medical device according to any of the embodiments could comprise at least one material selected from a group consisting of: polytetrafluoroethylene (PTFE), perfluoroalkoxy (PFA) and fluorinated ethylene propylene (FEP). It is furthermore conceivable that the material comprises a metal alloy, such as cobalt-chromium-molybdenum or titanium or stainless steel, or polyethylene, such as cross-linked polyethylene or gas sterilized polyethylene. The use of ceramic material is also conceivable, in the contacting surfaces or the entire medical device such as zirconium or zirconium dioxide ceramics or alumina ceramics. The part of the medical device in contact with human bone for fixation of the medical device to human bone could comprise a poorhouse structure which could be a porous micro or nano-structure adapted to promote the growth-in of human bone in the medical device for fixating the medical device. The porous structure could be achieved by applying a hydroxy-apatite (HA) coating, or a rough open-pored titanium coating, which could be produced by air plasma spraying, a combination comprising a rough open-pored titanium coating and a HA top layer is also conceivable. The contacting parts could be made of a self lubricated material such as a waxy polymer, such as PTFE, PFA, FEP, PE and UHMWPE, or a powder metallurgy material which could be infused with a lubricant, which preferably is a biocompatible lubricant such as a Hyaluronic acid derivate. It is also conceivable that the material of contacting parts or surfaces of the medical device herein is adapted to be constantly or intermittently lubricated. According to some embodiments the parts or portions of the medical device could comprise a combination of metal materials and/or carbon fibers and/or boron, a combination of metal and plastic materials, a combination of metal and carbon based material, a combination of carbon and plastic based material, a combination of flexible and stiff materials, a combination of elastic and less elastic materials, Corian or acrylic polymers.

According to the above mentioned embodiments the medical device is adapted to be inserted through a hole in the pelvic bone, however it is equally conceivable that the medical device according to any of the embodiment above is adapted to be inserted through a hole in the hip joint capsule or the femoral bone of the human patient.

## Claims

1. A medical device for implantation in a hip joint of a patient, the natural hip joint having a ball shaped caput femur integrated with a collum femur having a collum and caput center axis (CX), extending longitudinal along the collum and caput femur, in the center thereof, as the proximal part of the femoral bone with a convex hip joint surface towards the centre of the hip joint and a bowl shaped acetabulum as part of the pelvic bone with a concave hip joint surface towards the centre of the hip joint, the medical device comprising;
- an artificial caput femur (112), comprising a convex surface towards the centre of the hip joint, the convex surface having a center point adapted to be placed, when implanted, on the collum and caput center axis, and
- an elongated portion (113), adapted to be connected to a prosthetic spherical portion of said artificial convex caput femur and to be fixated inside of a bowl shaped acetabulum (110) as part of the pelvic bone of the human patient, wherein said artificial convex caput femur is adapted to have its spherical portion to be placed in contact with the inner surface of an prosthetic replacement for acetabulum to be movable in multiple directions replicating the natural ball and socket joint of the hip, wherein said artificial convex caput femur is further, when implanted, adapted to :
a. be fixated to at least the bowl shaped acetabulum of the pelvic bone of the human patient through said elongated portion fixation to the pelvic bone, and
b. be in movable connection with the comprised prosthetic acetabulum surface fixated to the femoral bone of the patient, comprising at least one extending portion (1823b) adapted to clasp said prosthetic spherical portion, wherein
c. said elongated portion comprises a restricting portion adapted to restrict the motion range of the artificial acetabulum in relation to said artificial convex caput femur, **characterized in that**
d. a larger portion of said restricting portion is placed, when implanted, dorsal to a vertical acetabulum plane (PX'Y') coinciding with the collum and caput center axis, to enable an advantageous motion range in relation to said artificial acetabulum.

2. The medical device according to claim 1, wherein said elongated portion comprises at least one recess (2203) adapted to receive a portion of said prosthetic artificial acetabulum, wherein said recess is adapted to be placed, when implanted, in at least in one of the following positions; frontal to the coronal pelvis plane (PXY), in the frontal quadrant (1812), in the proximal quadrant (1811), and in the distal quadrant (1813).

3. The medical device according to claim 2, wherein said recess is more than at least one of; 2 mm deep, 4 mm deep, 6 mm deep, and 8 mm deep.

4. The medical device according to any one of claims 1-3, wherein said elongated portion is adapted to receive said extending portion when implanted.

5. The medical device according to claim 4, wherein said adaptation comprises a bent portion of said elongated portion,

6. The medical device according to any one of claims 1 - 5, wherein a cross-section of said restricting portion of said elongated portion, perpendicular to the caput and collum center axis (CX), comprises a first distance and a second distance, wherein a center point of a line of said first distance intersects a center point of a line of said second distance, and wherein said first distance is shorter than said second distance, wherein said first distance is a cross-sectional distance of a narrow portion if said elongated portion, and wherein said second distance is a cross-sectional distance of a wide portion of said elongated portion.

7. The medical device according to any one of claims 1 - 6, wherein said restricting portion of said elongated portion is adapted to be at least one of; substantially aligned with said collum center axis and eccentrically placed in relation to said collum axis, when implanted, placed in at least one of; in the dorsal quadrant, in the proximal quadrant, and in the distal quadrant.

8. The medical device according to any one of claims 1 - 7, wherein a cross section of the restricting portion of the elongated portion, perpendicular to the collum center axis, is at least one of;
circular, polygonal, elliptical,
adapted to be placed such that adduction is restricted more degrees than flexion, when implanted,
adapted to be placed such that abduction is restricted more degrees than flexion, when implanted.
adapted to be placed such that adduction is restricted more degrees than extension, when implanted, and
adapted to be placed such that abduction is restricted more degrees than extension, when implanted.

9. The medical device according to any one of claims 1 - 8, comprising a prosthetic replacement for the acetabulum comprising at least one extending portion adapted to clasp said spherical portion, such that said spherical portion is restrained in said medical device, and wherein said prosthetic replacement for the acetabulum is adapted to be fixated to the femoral bone of the patient.

10. The medical device according to claim 9, wherein said prosthetic replacement for the acetabulum is adapted to receive said spherical portion being in connection with said medical device, wherein:
a) said inner surface comprises an equator line (1821), being the largest circular circumference of said inner contacting surface, being a surface adapted to be in contact with said spherical portion, and
b) said at least one extending portion passes beyond said equator line, such that the end portion of said contacting portion of said inner surface forms a circular extension line having a smaller circumference than said equator line,
c) said at least one extending portion circumferentially extends discontinuously along said equator line, such that a portion of said medical device can be placed between said extension line and said equator line, and
d) wherein said extension line is placed proximal to the equator line, when the medical device is implanted.

11. The medical device according to any one of claims 9 - 10, wherein said at least one extending portion extends circumferentially along said equator line, in at least one of the following position;
dorsal to the right-left axis of pelvis when being in the base position, dorsal to the coronal pelvis plane PXY and proximal to the horizontal pelvis PXZ plane when being in the base position,
dorsal to the coronal pelvis plane PXY and distal to the horizontal pelvis PXZ plane when being in the base position,
extends circumferentially along said equator line dorsal to the coronal pelvis plane PXY and proximal to the horizontal pelvis PXZ plane being at least one first extending portion, and at least one second extending portion extends dorsal to the coronal pelvis plane PXY and distal to the horizontal pelvis PXZ plane when being in the base position,
in the proximal quadrant (1811) of the equator line when being in the base position,
in the distal quadrant (1813) of the equator line when being in the base position,
in the distal and proximal quadrant thereof when being in the base position,
in the proximal and dorsal quadrant (1814) thereof when being in the base position,
in the distal and dorsal quadrant thereof when being in the base position, and
in the distal, dorsal and proximal quadrant thereof when being in the base position,

12. The medical device according to any one of claims 10 - 11, wherein at least a first portion of said prosthetic replacement for the acetabulum is an extending portion, extending beyond said circular equator line, and at least a second portion is a portion not extending beyond said circular equator line, wherein said second portion circumferentially extends at least in one of;
along at least ¼ of said circular equator line,
along at least 1/3 of said circular equator line, and
along at least ½ of said circular equator line.

13. The medical device according to any one of claims 10 - 12, wherein at least a first portion of said prosthetic replacement for the acetabulum is an extending portion, extending beyond said circular equator line, and at least a second portion is a portion not extending beyond said circular equator line, wherein said at least first portion circumferentially extends at least in one of;
along at least ¼ of said circular equator line,
along at least 1/3 of said circular equator line,
along at least ½ of said circular equator line,
along at least 1/10 of said circular equator line,
along at least 1/10 of said circular equator line, and said second portion circumferentially extends along at least ¼ of said circular equator line,
beyond said circular equator line with at least two first portions, wherein said first portions each circumferentially extends along at least 1/10 of said circular equator line, and said second portion circumferentially extends along at least ¼ of said circular equator line,
beyond said circular equator line with at least two first portions, and wherein one of said extending portions extends further than the other extending portion, and
circumferentially extends along said equator line between said two second portions with said at least two first portions, and wherein two second portions not extending beyond said circular equator line.

14. The medical device according to claims 9 - 13, wherein said extending portion adapted to clasp said spherical portion and said restricting portion of said elongated portion of said medical device is adapted to, when implanted in said base position, be placed such that their placement in relation to each other creates an advantageous motion range,
wherein said extending portion said restricting portion of said elongated portion is adapted to be at least one of; placed in relation to each other when implanted in said base position, such that adduction is restricted more degrees than flexion,
placed in relation to each other when implanted in said base position, such that abduction is restricted more degrees than flexion,
placed in relation to each other when implanted in said base position, such that adduction is restricted more degrees than extension, and
placed in relation to each other when implanted in said base position, such that abduction is restricted more degrees than extension.

## Patentansprüche

1. Medizinische Vorrichtung zur Implantation in ein Hüftgelenk eines Patienten, wobei das natürliche Hüftgelenk einen mit einem Oberschenkelknochenhals integrierten kugelförmigen Oberschenkelknochenkopf mit einer Hals- und Kopfmittelachse (CX) hat, die sich in Längsrichtung entlang dem Oberschenkelknochenhals und dem Oberschenkelknochenkopf in deren Mitte erstreckt, als der proximale Teil des Oberschenkelknochens mit einer konvexen Hüftgelenkfläche zu der Mitte des Hüftgelenks hin und einem pfannenförmigen Acetabulum als Teil des Hüftknochens mit einer konkaven Hüftgelenkfläche zu der Mitte des Hüftgelenks hin, wobei die medizinische Vorrichtung Folgendes umfasst:
- einen künstlichen Oberschenkelknochenkopf (112), der eine konvexe Fläche zu der Mitte des Hüftgelenks hin umfasst, wobei die konvexe Fläche einen Mittelpunkt hat, der geeignet ist, bei der Implantierung auf die Hals- und Kopfmittelachse platziert zu werden, und
- einen länglichen Abschnitt (113), der geeignet ist, mit einem kugelförmigen Protheseabschnitt des künstlichen konvexen Oberschenkelknochenkopfs verbunden und in einem pfannenförmigen Acetabulum (110) als Teil des Hüftknochens des menschlichen Patienten fixiert zu werden, wobei der künstliche konvexe Oberschenkelknochenkopf dazu geeignet ist, dass sein kugelförmiger Abschnitt in Kontakt mit der Innenfläche einer Acetabulumersatzprothese platziert wird, so dass er in mehreren Richtungen bewegbar ist und das natürliche Kugelgelenk der Hüfte repliziert, wobei der künstliche konvexe Oberschenkelknochenkopf bei der Implantierung ferner zu Folgendem geeignet ist:
a. durch die Fixierung des länglichen Abschnitts am Hüftknochen an mindestens dem pfannenförmigen Acetabulum des Hüftknochens des menschlichen Patienten fixiert zu sein und
b. mit der umfassten, am Oberschenkelknochen des Patienten fixierten Acetabulumprothesefläche in beweglicher Verbindung zu stehen, die mindestens einen verlängerten Abschnitt (1823b) umfasst, der zum Ergreifen des kugelförmigen Protheseabschnitts geeignet ist, wobei
c. der längliche Abschnitt einen Einschränkungsabschnitt umfasst, der geeignet ist, den Bewegungsspielraum des künstlichen Acetabulums bezüglich des künstlichen konvexen Oberschenkelknochenkopfs zu begrenzen, **dadurch gekennzeichnet, dass**
d. ein größerer Abschnitt des Einschränkungsabschnitts bei der Implantierung dorsal zu einer mit der Hals- und Kopfmittelachse zusammenfallenden vertikalen Acetabulumebene (PX'Y') platziert ist, um einen vorteilhaften Bewegungsspielraum bezüglich des künstlichen Acetabulums zu ermöglichen.

2. Medizinische Vorrichtung nach Anspruch 1, wobei der längliche Abschnitt mindestens eine Aussparung (2203) umfasst, die zur Aufnahme eines Abschnitts der künstlichen Acetabulumprothese geeignet ist, wobei die Aussparung geeignet ist, bei der Implantierung in mindestens eine der folgenden Positionen platziert zu werden: frontal zu der Frontalebene (PXY), im vorderen Quadranten (1812), im proximalen Quadranten (1811) und im distalen Quadranten (1813).

3. Medizinische Vorrichtung nach Anspruch 2, wobei die Aussparung mehr als 2mm tief und/oder 4 mm tief und/oder 6 mm tief und/oder 8 mm tief ist.

4. Medizinische Vorrichtung nach einem der Ansprüche 1 - 3, wobei der längliche Abschnitt bei der Implantierung zur Aufnahme des verlängerten Abschnitts geeignet ist.

5. Medizinische Vorrichtung nach Anspruch 4, wobei die Anpassung einen gebogenen Abschnitt des länglichen Abschnitts umfasst.

6. Medizinische Vorrichtung nach einem der Ansprüche 1 - 5, wobei ein senkrecht zur Kopf- und Halsmittelachse (CX) verlaufender Querschnitt des Einschränkungsabschnitts des länglichen Abschnitts einen ersten Abstand und einen zweiten Abstand umfasst, wobei ein Mittelpunkt einer Linie des ersten Abstands einen Mittelpunkt einer Linie des zweiten Abstands schneidet und wobei der erste Abstand kürzer ist als der zweite Abstand, wobei der erste Abstand ein Querschnittsabstand eines schmalen Abschnitts des länglichen Abschnitts ist und wobei der zweite Abstand ein Querschnittsabstand eines breiten Abschnitts des länglichen Abschnitts ist.

7. Medizinische Vorrichtung nach einem der Ansprüche 1 - 6, wobei der Einschränkungsabschnitt des länglichen Abschnitts geeignet ist, bei der Implantierung im Wesentlichen auf die Halsmittelachse ausgerichtet zu sein und/oder exzentrisch zu der Halsachse platziert zu sein und im dorsalen Quadranten und/oder im proximalen Quadranten und/oder im distalen Quadranten platziert zu sein.

8. Medizinische Vorrichtung nach einem der Ansprüche 1 - 7, wobei ein senkrecht zu der Halsmittelachse verlaufender Querschnitt des Einschränkungsabschnitts des länglichen Abschnitts
kreisförmig, polygonal, elliptisch ist und/oder dazu geeignet ist, so platziert zu sein, dass die Adduktion bei der Implantierung um mehr Grade eingeschränkt ist als die Flexion, und/oder dazu geeignet ist, so platziert zu sein, dass die Abduktion bei der Implantierung um mehr Grade eingeschränkt ist als die Flexion, und/oder dazu geeignet ist, so platziert zu sein, dass die Adduktion bei der Implantierung um mehr Grade eingeschränkt ist als die Extension, und/oder dazu geeignet ist, so platziert zu sein, dass die Abduktion bei der Implantierung um mehr Grade eingeschränkt ist als die Extension.

9. Medizinische Vorrichtung nach einem der Ansprüche 1 - 8, umfassend eine Ersatzprothese für das Acetabulum, die mindestens einen verlängerten Abschnitt umfasst, der zum Ergreifen des kugelförmigen Abschnitts geeignet ist, so dass der kugelförmige Abschnitt in der medizinischen Vorrichtung gehalten wird, und wobei die Ersatzprothese für das Acetabulum zur Fixierung an dem Oberschenkelknochen des Patienten geeignet ist.

10. Medizinische Vorrichtung nach Anspruch 9, wobei die Ersatzprothese für das Acetabulum zur Aufnahme des kugelförmigen Abschnitts geeignet ist, der mit der medizinischen Vorrichtung in Verbindung steht, wobei:
a) die Innenfläche eine Äquatorlinie (1821) umfasst, die der größte Kreisumfang der inneren Kontaktfläche ist, die eine Fläche ist, die dazu geeignet ist, mit dem kugelförmigen Abschnitt in Kontakt zu sein, und
b) der mindestens eine verlängerte Abschnitt über die Äquatorlinie hinausgeht, so dass der Endabschnitt des Kontaktabschnitts der Innenfläche eine kreisförmige Verlängerungslinie mit einem kleineren Umfang als die Äquatorlinie bildet,
c) sich der mindestens eine verlängerte Abschnitt umfangsmäßig diskontinuierlich entlang der Äquatorlinie erstreckt, so dass ein Abschnitt der medizinischen Vorrichtung zwischen die Verlängerungslinie und die Äquatorlinie platziert werden kann, und
d) wobei die Verlängerungslinie proximal zu der Äquatorlinie platziert ist, wenn die medizinische Vorrichtung implantiert ist.

11. Medizinische Vorrichtung nach einem der Ansprüche 9 - 10, wobei sich der mindestens eine verlängerte Abschnitt in mindestens einer der folgenden Positionen umfangsmäßig entlang der Äquatorlinie erstreckt:
dorsal zu der Rechts-Links-Achse der Hüfte, wenn in der Basisposition,
dorsal zu der Frontalebene PXY und proximal zu der horizontalen Hüftebene PXZ, wenn in der Basisposition,
dorsal zu der Frontalebene PXY und distal zu der horizontalen Hüftebene PXZ, wenn in der Basisposition,
erstreckt sich umfangsmäßig entlang der Äquatorlinie dorsal zu der Frontalebene PXY und proximal zu der horizontalen Hüftebene PXZ, wobei er mindestens ein erster verlängerter Abschnitt ist, und sich mindestens ein zweiter verlängerter Abschnitt dorsal zu der Frontalebene PXY und distal zu der horizontalen Hüftebene PXZ erstreckt, wenn in der Basisposition,
im proximalen Quadranten (1811) der Äquatorlinie, wenn in der Basisposition,
im distalen Quadranten (1813) der Äquatorlinie, wenn in der Basisposition,
im distalen und proximalen Quadranten davon, wenn in der Basisposition,
im proximalen und dorsalen Quadranten (1814) davon, wenn in der Basisposition,
im distalen und dorsalen Quadranten davon, wenn in der Basisposition, und
im distalen, dorsalen und proximalen Quadranten davon, wenn in der Basisposition.

12. Medizinische Vorrichtung nach einem der Ansprüche 10 - 11, wobei mindestens ein erster Abschnitt der Ersatzprothese für das Acetabulum ein verlängerter Abschnitt ist, der sich über die kreisförmige Äquatorlinie hinaus erstreckt, und mindestens ein zweiter Abschnitt ein Abschnitt ist, der sich nicht über die kreisförmige Äquatorlinie hinaus erstreckt, wobei sich der zweite Abschnitt umfangsmäßig wie folgt erstreckt:
entlang mindestens 1/4 der kreisförmigen Äquatorlinie und/oder
entlang mindestens 1/3 der kreisförmigen Äquatorlinie und/oder
entlang mindestens 1/2 der kreisförmigen Äquatorlinie.

13. Medizinische Vorrichtung nach einem der Ansprüche 10 - 12, wobei mindestens ein erster Abschnitt der Ersatzprothese für das Acetabulum ein verlängerter Abschnitt ist, der sich über die kreisförmige Äquatorlinie hinaus erstreckt, und mindestens ein zweiter Abschnitt ein Abschnitt ist, der sich nicht über die kreisförmige Äquatorlinie hinaus erstreckt, wobei sich der mindestens erste Abschnitt umfangsmäßig wie folgt erstreckt:
entlang mindestens 1/4 der kreisförmigen Äquatorlinie und/oder
entlang mindestens 1/3 der kreisförmigen Äquatorlinie und/oder
entlang mindestens 1/2 der kreisförmigen Äquatorlinie und/oder
entlang mindestens 1/10 der kreisförmigen Äquatorlinie und/oder
entlang mindestens 1/10 der kreisförmigen Äquatorlinie und sich der zweite Abschnitt umfangsmäßig entlang mindestens 1/4 der kreisförmigen Äquatorlinie erstreckt und/oder
über die kreisförmige Äquatorlinie hinaus mit mindestens zwei ersten Abschnitten, wobei sich die ersten Abschnitte jeweils umfangsmäßig entlang mindestens 1/10 der kreisförmigen Äquatorlinie erstrecken und sich der zweite Abschnitt umfangsmäßig entlang mindestens 1/4 der kreisförmigen Äquatorlinie erstreckt, und/oder
über die kreisförmige Äquatorlinie hinaus mit mindestens zwei ersten Abschnitten, und wobei sich einer der verlängerten Abschnitte weiter als der andere verlängerte Abschnitt erstreckt, und/oder sich umfangsmäßig entlang der Äquatorlinie zwischen den beiden zweiten Abschnitten mit den mindestens zwei ersten Abschnitten erstreckt, und wobei sich zwei zweite Abschnitte nicht über die kreisförmige Äquatorlinie hinaus erstrecken.

14. Medizinische Vorrichtung nach einem der Ansprüche 9 - 13, wobei der verlängerte Abschnitt, der dazu geeignet ist, den kugelförmigen Abschnitt und den Einschränkungsabschnitt des länglichen Abschnitts der medizinischen Vorrichtung zu ergreifen, geeignet ist, bei Implantierung in der Basisposition, so platziert zu sein, dass ihre Platzierung bezüglich einander einen vorteilhaften Bewegungsspielraum erzeugt,
wobei der verlängerte Abschnitt und der Einschränkungsabschnitt des länglichen Abschnitts dazu geeignet sind,
bei Implantierung in der Basisposition so bezüglich einander platziert zu sein, dass die Adduktion um mehr Grade eingeschränkt ist als die Flexion, und/oder
bei Implantierung in der Basisposition so bezüglich einander platziert zu sein, dass die Abduktion um mehr Grade eingeschränkt ist als die Flexion, und/oder
bei Implantierung in der Basisposition so bezüglich einander platziert zu sein, dass die Adduktion um mehr Grade eingeschränkt ist als die Extension, und/oder
bei Implantierung in der Basisposition so bezüglich einander platziert zu sein, dass die Abduktion um mehr Grade eingeschränkt ist als die Extension.

## Revendications

1. Dispositif médical destiné à être implanté dans l'articulation de la hanche d'un patient, l'articulation de la hanche naturelle comportant une tête de fémur en forme de sphère intégrée à un col de fémur présentant un axe (CX) central de col et de tête, qui s'étend longitudinalement le long du col et de la tête de fémur, au centre de ceux-ci, constituant la partie proximale de l'os fémoral présentant une surface d'articulation de hanche convexe en direction du centre de l'articulation de la hanche ainsi qu'un acétabulum en forme de cuvette constituant la partie de l'os pelvien présentant une surface d'articulation de la hanche concave en direction du centre de l'articulation de la hanche, le dispositif médical comportant :
- une tête de fémur (112) artificielle, comportant une surface convexe en direction du centre de l'articulation de la hanche, la surface convexe présentant un point central conçu pour être placé, lorsque le dispositif est implanté, sur l'axe central du col et de la tête, et
- une partie (113) allongée, conçue pour être reliée à une partie sphérique prothétique de ladite tête de fémur convexe artificielle et pour être fixée à l'intérieur d'un acétabulum (110) en forme de cuvette constituant l'os pelvien du patient humain, ladite tête de fémur convexe artificielle étant conçue pour avoir sa partie sphérique placée en contact avec la surface intérieure d'un substitut prothétique d'acétabulum destiné à être mobile dans de multiples directions pour reproduire l'articulation à rotule naturelle de la hanche, ladite tête de fémur convexe artificielle étant en outre, lorsque le dispositif est implanté, conçue pour :
a. être fixée à au moins l'acétabulum en forme de cuvette de l'os pelvien du patient humain au moyen de ladite fixation de la partie allongée à l'os pelvien, et
b. être en liaison mobile avec la surface d'acétabulum prothétique comprise fixée à l'os fémoral du patient, comprenant au moins une partie (1823b) d'extension conçue pour enserrer ladite partie sphérique prothétique, dans lequel
c. ladite partie allongée comprend une partie restrictive conçue pour limiter la plage de mouvement de l'acétabulum artificiel par rapport à ladite tête de fémur convexe artificielle, **caractérisé en ce que**
d. une partie plus grande de ladite partie restrictive est placée, lorsque le dispositif est implanté, de façon dorsale à un plan (PX'Y') d'acétabulum vertical qui coïncide avec l'axe central du col et de la tête, afin de permettre une plage de mouvement avantageuse par rapport audit acétabulum artificiel.

2. Dispositif médical selon la revendication 1, dans lequel ladite partie allongée comprend au moins un évidement (2203) conçu pour recevoir une partie dudit acétabulum artificiel prothétique, ledit évidement étant conçu pour être placé, lorsque le dispositif est implanté, dans au moins l'une des positions suivantes : en position frontale par rapport au plan (PXY) pelvien coronal, dans le quadrant (1812) frontal, dans le quadrant (1811) proximal, et dans le quadrant (1813) distal.

3. Dispositif médical selon la revendication 2, dans lequel ledit évidement est supérieur à au moins l'une des épaisseurs suivantes : 2 mm, 4 mm, 6 mm, et 8 mm.

4. Dispositif médical selon l'une quelconque des revendications 1 à 3, dans lequel ladite partie allongée est conçue pour recevoir ladite partie d'extension lorsque le dispositif est implanté.

5. Dispositif médical selon la revendication 4, dans lequel ladite conception comprend une partie courbe de ladite partie allongée.

6. Dispositif médical selon l'une quelconque des revendications 1 à 5, dans lequel une section transversale de ladite partie restrictive de ladite partie allongée, perpendiculaire à l'axe (CX) central de la tête et du col, comprend une première distance et une deuxième distance, un point central d'une ligne de ladite première distance croisant un point central d'une ligne de ladite deuxième distance, et dans lequel ladite première distance est plus courte que ladite deuxième distance, ladite première distance étant une distance en coupe transversale d'une partie étroite de ladite partie allongée, et dans lequel ladite deuxième distance est une distance en coupe transversale d'une partie large de ladite partie allongée.

7. Dispositif médical selon l'une quelconque des revendications 1 à 6, dans lequel ladite partie restrictive de ladite partie allongée est conçue pour être au moins dans l'une des situations suivantes : sensiblement alignée avec ledit axe central de col et placée de manière excentrique par rapport audit axe de col, lorsque le dispositif est implanté, placée dans au moins l'une des positions suivantes : dans le quadrant dorsal, dans le quadrant proximal, et dans le quadrant distal.

8. Dispositif médical selon l'une quelconque des revendications 1 à 7, dans lequel une section transversale de la partie restrictive de la partie allongée, perpendiculaire à l'axe central du col, est au moins l'une des possibilités suivantes :
circulaire, polygonale, elliptique,
conçue pour être placée de telle sorte que l'adduction soit limitée dans une plus grande mesure que la flexion, lorsque le dispositif est implanté,
conçue pour être placée de telle sorte que l'abduction soit limitée dans une plus grande mesure que la flexion, lorsque le dispositif est implanté,
conçue pour être placée de telle sorte que l'adduction soit limitée dans une plus grande mesure que l'extension, lorsque le dispositif est implanté, et
conçue pour être placée de telle sorte que l'abduction soit limitée dans une plus grande mesure que l'extension, lorsque le dispositif est implanté.

9. Dispositif médical selon l'une quelconque des revendications 1 à 8, comprenant un substitut prothétique d'acétabulum comprenant au moins une partie d'extension conçue pour enserrer ladite partie sphérique, de telle sorte que ladite partie sphérique soit retenue dans ledit dispositif médical, et dans lequel ledit substitut prothétique d'acétabulum est conçu pour être fixé à l'os fémoral du patient.

10. Dispositif médical selon la revendication 9, dans lequel ledit substitut prothétique d'acétabulum est conçu pour recevoir ladite partie sphérique qui est en liaison avec le dispositif médical, dans lequel :
a) ladite surface intérieure comprend une ligne (1821) équinoxiale, qui constitue la plus grande circonférence circulaire de ladite surface de contact intérieure, étant une surface conçue pour être en contact avec ladite partie sphérique, et
b) ladite au moins une partie d'extension passe au-delà de ladite ligne équinoxiale, de telle sorte que la partie d'extrémité de ladite partie de contact de ladite surface intérieure forme une ligne d'extension circulaire présentant une circonférence plus petite que ladite ligne équinoxiale,
c) ladite au moins une partie d'extension s'étend circonférentiellement de manière discontinue le long de la ligne équinoxiale, de telle sorte qu'une partie dudit dispositif médical peut être placée entre ladite ligne d'extension et ladite ligne équinoxiale, et
d) dans lequel ladite ligne d'extension est placée proximalement à la ligne équinoxiale, lorsque le dispositif médical est implanté.

11. Dispositif médical selon l'une quelconque des revendications 9 à 10, dans lequel ladite au moins une partie d'extension s'étend circonférentiellement le long de ladite ligne équinoxiale, dans au moins une des positions suivantes :
dorsale à l'axe droite-gauche du pelvis lorsqu'elle se trouve dans la position de base,
dorsale au plan PXY pelvien coronal et proximale au plan PXZ pelvien horizontal lorsqu'elle se trouve dans la position de base,
dorsale au plan PXY pelvien coronal et distale au plan PXZ pelvien horizontal lorsqu'elle se trouve dans la position de base,
s'étend circonférentiellement le long de ladite ligne équinoxiale de façon dorsale au plan PXY pelvien coronal et de manière proximale au plan PXZ pelvien horizontal qui constitue au moins une première partie d'extension, et au moins une deuxième partie d'extension s'étend de façon dorsale au plan PXY pelvien coronal et distale au plan PXZ pelvien horizontal lorsqu'elle se trouve dans la position de base,
dans le quadrant (1811) proximal de la ligne équinoxiale lorsqu'elle se trouve dans la position de base,
dans le quadrant (1813) distal de la ligne équinoxiale lorsqu'elle se trouve dans la position de base,
dans le quadrant distal et proximal de celle-ci lorsqu'elle se trouve dans la position de base,
dans le quadrant (1814) proximal et dorsal de celle-ci lorsqu'elle se trouve dans la position de base,
dans le quadrant distal et dorsal de celle-ci lorsqu'elle se trouve dans la position de base, et
dans le quadrant distal, dorsal et proximal de celle-ci lorsqu'elle se trouve dans la position de base.

12. Dispositif médical selon l'une quelconque des revendications 10 à 11, dans lequel au moins une première partie dudit substitut prothétique d'acétabulum constitue une partie d'extension, qui s'étend au-delà de ladite ligne équinoxiale circulaire, et au moins une deuxième partie constitue une partie qui ne s'étend pas au-delà de ladite ligne équinoxiale circulaire, ladite deuxième partie s'étendant circonférentiellement d'au moins une des façons suivantes :
le long d'au moins 1/4 de ladite ligne équinoxiale circulaire,
le long d'au moins 1/3 de ladite ligne équinoxiale circulaire, et
le long d'au moins 1/2 de ladite ligne équinoxiale circulaire.

13. Dispositif médical selon l'une quelconque des revendications 10 à 12, dans lequel au moins une première partie dudit substitut prothétique d'acétabulum constitue une partie d'extension, qui s'étend au-delà de ladite ligne équinoxiale circulaire, et au moins une deuxième partie constitue une partie qui ne s'étend pas au-delà de ladite ligne équinoxiale circulaire, ladite au moins une partie s'étendant circonférentiellement d'au moins une des façons suivantes:
le long d'au moins 1/4 de ladite ligne équinoxiale circulaire,
le long d'au moins 1/3 de ladite ligne équinoxiale circulaire,
le long d'au moins 1/2 de ladite ligne équinoxiale circulaire,
le long d'au moins 1/10 de ladite ligne équinoxiale circulaire,
le long d'au moins 1/10 de ladite ligne équinoxiale circulaire, et ladite deuxième partie s'étend circonférentiellement le long d'au moins 1/4 de ladite ligne équinoxiale circulaire,
au-delà de ladite ligne équinoxiale circulaire avec au moins deux premières parties, dans lequel lesdites premières parties s'étendent chacune circonférentiellement le long d'au moins 1/10 de ladite ligne équinoxiale circulaire, et ladite deuxième partie s'étend circonférentiellement le long d'au moins 1/4 de ladite ligne équinoxiale circulaire,
au-delà de ladite ligne équinoxiale circulaire avec au moins deux premières parties, et dans lequel une desdites parties d'extension s'étend plus loin que l'autre partie d'extension, et
s'étend circonférentiellement le long de ladite ligne équinoxiale entre lesdites deuxièmes parties avec lesdites au moins deux premières parties, et dans lequel deux deuxièmes parties ne s'étendent pas au-delà de ladite ligne équinoxiale circulaire.

14. Dispositif médical selon les revendications 9 à 13, dans lequel ladite partie d'extension est conçue pour enserrer ladite partie sphérique et ladite partie restrictive de ladite partie allongée dudit dispositif médical est conçue, lorsque le dispositif est implanté dans ladite position de base, pour être placée de telle sorte que leur placement l'une par rapport à l'autre crée une plage de mouvement avantageuse,
dans lequel ladite partie restrictive et ladite partie d'extension de ladite partie allongée sont conçues pour être au moins : soit placées l'une par rapport à l'autre lorsqu'elles sont implantées dans ladite position de base, de telle sorte que l'adduction soit limitée dans une plus grande mesure que la flexion,
soit placées l'une par rapport à l'autre lorsqu'elles sont implantées dans ladite position de base, de telle sorte que l'abduction soit limitée dans une plus grande mesure que la flexion,
soit placées l'une par rapport à l'autre lorsqu'elles sont implantées dans ladite position de base, de telle sorte que l'adduction soit limitée dans une plus grande mesure que l'extension, et
soit placées l'une par rapport à l'autre lorsqu'elles sont implantées dans ladite position de base, de telle sorte que l'abduction soit limitée dans une plus grande mesure que l'extension.
